(19) Europäisches Patentamt / European Patent Office / Office européen des brevets

(11) **EP 3 033 042 B1**

(12) **EUROPÄISCHE PATENTSCHRIFT**

(45) Veröffentlichungstag und Bekanntmachung des Hinweises auf die Patenterteilung:
**09.06.2021 Patentblatt 2021/23**

(21) Anmeldenummer: **14747635.2**

(22) Anmeldetag: **04.08.2014**

(51) Int Cl.:
*A61F 2/16* *(2006.01)*          *G02B 3/06* *(2006.01)*
*G02C 7/04* *(2006.01)*

(86) Internationale Anmeldenummer:
**PCT/EP2014/066695**

(87) Internationale Veröffentlichungsnummer:
**WO 2015/022215 (19.02.2015 Gazette 2015/07)**

(54) **AUGENLINSE MIT EINEM TORISCH BRECHENDEN OBERFLÄCHENPROFIL UND EINER IN RADIALER RICHTUNG GESTUFTEN OBERFLÄCHENSTRUKTUR**

EYE LENS HAVING A TORIC REFRACTIVE SURFACE PROFILE AND A SURFACE STRUCTURE THAT IS STEPPED IN A RADIAL DIRECTION

LENTILLE INTRAOCULAIRE PRÉSENTANT UN PROFIL DE SURFACE À RÉFRACTION TORIQUE ET UNE STRUCTURE DE SURFACE ÉCHELONNÉE EN DIRECTION RADIALE

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **13.08.2013 DE 102013216020**

(43) Veröffentlichungstag der Anmeldung:
**22.06.2016 Patentblatt 2016/25**

(73) Patentinhaber: **Carl Zeiss Meditec AG**
**07745 Jena (DE)**

(72) Erfinder: **GERLACH, Mario**
**16548 Glienicke-Nordbahn (DE)**

(74) Vertreter: **Hofstetter, Schurack & Partner**
**Patent- und Rechtsanwaltskanzlei**
**PartG mbB**
**Balanstrasse 57**
**81541 München (DE)**

(56) Entgegenhaltungen:
WO-A1-2012/083143     WO-A1-2014/111831
DE-A1-102005 028 933     US-A1- 2003 033 013
US-A1- 2009 164 008     US-A1- 2011 149 236

**Beschreibung**

Die Erfindung betrifft eine Augenlinse mit einem optischen Teil, der eine in Richtung einer optischen Hauptachse der Augenlinse betrachtet erste optische Seite und eine gegenüberliegende zweite optische Seite aufweist. Auf zumindest einer der beiden Seiten ist ein torisch brechendes Oberflächenprofil ausgebildet.

Stand der Technik

Torische Augenlinsen insbesondere Intraokularlinsen, sind aus dem Stand der Technik bekannt. Beispielsweise ist eine torische Intraokularlinse aus der DE 10 2005 028 933 A1 bekannt. Durch torische Intraokularlinsen wird insbesondere der Sehfehler Astigmatismus korrigiert.

Die bekannten torischen Linsen weisen jedoch ein relativ großes Volumen in ihrem insbesondere optischen Teil auf. Dies wird durch die erforderliche Mittendicke des optischen Teils im steilen Hauptmeridian erzeugt. Daher sind Linsen mit hoher sphärischer Brechkraft und Zylinderwerten nur durch größere Inzissionsweiten zu implantieren, oder der Durchmesser des optischen Teils muss reduziert werden.

Aus der US 2009/164008 A1 ist eine Intraokularlinse bekannt, die ein torisches Oberflächenprofil und ein gestuftes Oberflächenprofil aufweist.

Darstellung der Erfindung

Es ist Aufgabe der vorliegenden Erfindung, eine Augenlinse mit einem torisch brechenden Oberflächenprofil zu schaffen, welche in ihrem Linsenvolumen, insbesondere im optischen Teil verkleinert ist.

Diese Aufgabe wird durch eine Augenlinse, wie sie durch die Merkmale des Anspruchs 1 ausgebildet ist, gelöst.

Eine erfindungsgemäße Augenlinse umfasst einen optischen Teil, mit welchem die Abbildungseigenschaften der Augenlinse charakterisiert sind. Dieser optische Teil weist eine in Richtung einer optischen Hauptachse der Augenlinse betrachtet erste optische Seite, die eine Vorderseite und eine Rückseite sein kann, und eine der ersten Seite am optischen Teil axial gegenüberliegende zweite optische Seite auf, die abhängig von der ersten Seite dann eine Rückseite oder Vorderseite sein kann. Die optische Hauptachse steht senkrecht auf einer axial zwischen der ersten und der zweiten Seite, insbesondere der Vorderseite und der Rückseite, liegenden Ebene der Augenlinse, insbesondere des optischen Teils, so dass die Seiten gegenüberliegend zu dieser Ebene entlang der Hauptachse betrachtet angeordnet sind. Zumindest auf einer der beiden Seiten und somit zumindest auf der Vorderseite oder der Rückseite ist ein zur optischen Abbildungseigenschaft der Augenlinse beitragendes, torisch brechendes Oberflächenprofil ausgebildet. Ein torisch brechendes Oberflächenprofil charakterisiert sich insbesondere dadurch, dass zwei senkrecht aufeinander stehende Hauptmeridiane unterschiedliche Radien, insbesondere Scheitelradien, aufweisen, und somit auch die Krümmungen, insbesondere Scheitelkrümmungen, unterschiedlich sind. Dadurch wird insbesondere in azimutaler Richtung und somit in Umlaufrichtung um die optische Hauptachse eine Variation der Brechkraft der Augenlinse erreicht. Durch die spezifische Ausgestaltung der Hauptmeridiane variiert diese Brechkraft in azimutaler Richtung insbesondere entsprechend einem Kurvenverlauf, der zwischen einem Anfang und einem Ende in azimutaler Richtung betrachtet zumindest mit Zwischenminima ausgebildet ist.

Ein wesentlicher Gedanke der Erfindung ist darin zu sehen, dass die Augenlinse zusätzlich zum torisch brechenden Oberflächenprofil eine in radialer Richtung des optischen Teils gestufte Oberflächenstruktur aufweist, die auf zumindest einer der beiden Seiten ausgebildet ist und welche insbesondere in azimutaler Richtung zumindest teilweise umlaufend ausgebildet ist. Durch eine derartige insbesondere sägezahnartig gestufte Oberflächenstruktur wird eine optische Ausgestaltung geschaffen, welche es ermöglicht, dass bei auch torischen Augenlinsen zumindest gleichbleibende optische Abbildungseigenschaften einhergehen, jedoch das Linsenvolumen und somit auch das Volumen des optischen Teils wesentlich reduziert werden kann. Insbesondere ist durch diese Ausgestaltung mit einer zusätzlichen sägezahnartig gestuften Oberflächenstruktur die Ausdehnung des optischen Teils in axialer Richtung deutlich reduziert und somit insbesondere auch die Mittendicke wesentlich reduziert.

Durch eine derartige Ausgestaltung wird somit auch eine Augenlinse geschaffen, die bei einer Ausgestaltung als Intraokularlinse eine wesentlich verbesserte Kleinschnitttauglichkeit aufweist. Dies bedeutet, dass beim Einsetzen der Intraokularlinse in das Auge ein deutlich kleinerer Schnitt im Auge erforderlich ist, um dadurch die Intraokularlinse in das Auge einbringen zu können.

Die gestufte Oberflächenstruktur ist in azimutaler Richtung und somit um die Achse betrachtet zumindest teilweise umlaufend ausgebildet.

Gerade mit einer sägezahnartig gestuften Oberflächenstruktur wird somit insbesondere auch eine Fresnellierung zumindest einer Seite ausgebildet. Insbesondere ist mit einer Fresnellierung im Kontext der Erfindung eine Ausgestaltung zu verstehen, bei welcher ein Phasenhub zwischen zwei in radialer Richtung benachbarten und aneinander angrenzenden Zonen und somit auch eine dazwischen gebildete Stufe $n\lambda$, mit $n > 1$, insbesondere $\geq 10$, insbesondere

$\geq$ 15, ist.

Der Sehfehler Astigmatismus ist dadurch charakterisiert, dass sich die wirksamen Brechkräfte zweier unterschiedlicher Azimuten unterscheiden und dadurch eine punktförmige Abbildung eines Objektpunkts verhindert wird. In der Regel stehen die beiden Azimute senkrecht zueinander und stellen dadurch Hauptmeridiane dar. Die primäre Quelle für den Astigmatismus des menschlichen Auges ist eine gestörte Rotationssymmetrie der menschlichen Hornhaut. Hierbei unterscheiden sich in zwei senkrecht zueinander stehenden Richtungen die Krümmungsradien. Gerade im Fall einer Kataraktoperation kann die Korrektur des kornealen Astigmatismus von einer Intraokularlinse übernommen werden und dem Patienten ein brillenfreies Sehen ermöglicht werden.

Insbesondere stehen Hauptmeridiane des torisch brechenden Oberflächenprofils senkrecht aufeinander in sind somit azimutal entsprechend 90° versetzt zueinander.

Die Augenlinse ist insbesondere eine Intraokularlinse.

Vorzugsweise ist vorgesehen, dass das torisch brechende Oberflächenprofil und die gestufte Oberflächenstruktur auf einer gemeinsamen Seite des optischen Teils ausgebildet und überlagert sind. Dies kann die Vorderseite oder aber auch die Rückseite sein. Durch eine derartige Ausgestaltung kann die Lage der beiden unterschiedlichen separaten Oberflächentopographien sehr genau erreicht werden.

Es kann auch vorgesehen sein, dass das torisch brechende Oberflächenprofil auf einer Seite des optischen Teils und die gestufte Oberflächenstruktur auf der anderen Seite des optischen Teils ausgebildet sind. Dabei kann es vorgesehen sein, dass das torisch brechende Oberflächenprofil auf der Vorderseite und die gestufte Oberflächenstruktur auf der Rückseite ausgebildet sind oder das torisch brechende Oberflächenprofil auf der Rückseite und die gestufte Oberflächenstruktur auf der Vorderseite ausgebildet sind. Bei einer derartigen Ausgestaltung sind die individuellen separaten Oberflächentopographien in ihrer Kontur sehr genau herstellbar, so dass die Einzelstrukturen dann sehr präzise Abbildungseigenschaften ermöglichen.

Es kann auch vorgesehen sein, dass sowohl auf der Vorderseite als auch auf der Rückseite jeweils sowohl ein torisch brechendes Oberflächenprofil und überlagert eine gestufte Oberflächenstruktur ausgebildet sind. Die jeweiligen individuellen separaten Oberflächentopographien sind dann vorzugsweise so ausgebildet, dass sie in Summe jeweils den gesamten Brechkraftanteil, den sie zur Gesamtbrechkraft der Augenlinse beitragen, ergeben. Im Vergleich zu einer Aufbringung einer Oberflächentopographie auf lediglich einer der beiden Seiten erfolgt durch diese beidseitige Aufbringung eine Aufteilung der optischen Wirkung bezüglich der Brechkraftverteilung.

Es ist vorgesehen, dass ein radialer Kurvenverlauf einer Referenzverbindungskurve, die auf einer Seite des optischen Teils ausgebildete Stufen jeweils, bezogen auf die individuellen axialen Längen dieser Stufen und somit der Stufenhöhen, auf im Vergleich der Längen der Stufen gleicher axialer Längenverhältnislage schneidet, zumindest abschnittsweise einem an einer auf der optischen Hauptachse senkrecht stehenden Symmetrieebene der Augenlinse gespiegelten Konturenverlauf der Oberfläche der gegenüberliegenden anderen Seite entspricht. Dies bedeutet, dass in radialer Richtung betrachtet die gestufte Oberflächenstruktur mehrere Stufen mit individuellen Stufenhöhen aufweisen, die insbesondere mit größer werdendem Radius kleiner werden. Aufgrund dieser Variation der Stufenhöhen ist die Referenzverbindungskurve nicht jeweils durch einen Absolutwert einer Länge einer Stufenhöhe zu schneiden, sondern in einem prozentualen Längenanteil der jeweiligen Stufe. Dies bedeutet, dass beispielsweise bei einer ersten Stufe, die eine Stufenhöhe a aufweist und bei einer nachfolgenden Stufe, die eine dazu unterschiedliche Stufenhöhe b aufweist, die Referenzverbindungskurve beide Stufenhöhen in einem prozentual gleichen Längenanteil schneidet. Dies bedeutet, dass dann die Referenzverbindungskurve die erste Stufenhöhe beispielsweise bei 50 % der Stufenhöhe a und die zweite Stufe ebenfalls bei 50 % der Stufenhöhe b schneidet. Dieser spezifische prozentuale Wert wird dann auch bei allen anderen Stufenhöhen zugrunde gelegt. Dies bedeutet, dass dann bei diesem spezifischen Ausführungsbeispiel die Referenzverbindungskurve alle Stufenhöhen auf der Hälfte ihrer Längen schneidet. Es kann auch vorgesehen sein, dass die Referenzverbindungskurve die Stufen in einen gleichen anderen prozentualen Längenanteilswert schneidet. Dieser kann zwischen 0 % und 100 % variieren. Somit kann die Referenzverbindungskurve die Stufenhöhen auch am Stufenboden oder auch an der Stufenspitze schneiden. Gerade bei einem Schneiden beziehungsweise einem Verbinden der Stufenspitzen der einzelnen Stufen wird dann eine Einhüllende zugrunde gelegt. Bei einer derartigen Ausgestaltung ist es dann somit vorteilhaft, dass ein radialer Konturenverlauf einer Einhüllenden, die auf einer Seite ausgebildete Stufenspitzen von die gestufte Oberflächenstruktur bildenden Stufen verbindet, zumindest abschnittsweise einem an einer auf der optischen Hauptachse senkrecht stehenden Symmetrieebene der Augenlinse gespiegelten Konturenverlauf der Oberfläche der gegenüberliegenden anderen Seite entspricht.

Alle vorher gemachten und nachfolgend noch gemachten Angaben im Hinblick auf Gleichheiten zwischen Parametern, Parameterwerten und Topographieangaben sind im Rahmen von Abweichungen, die durch Maßtoleranzen, Fertigungstoleranzen und Messtoleranzen entstehen, ebenfalls noch als von der Erfindung umfasst anzusehen. In dem Zusammenhang sind auch entsprechende Erläuterungen, die sich auf im Wesentlichen gleiche Angaben beziehen, ebenfalls darunter zu verstehen.

Vorzugsweise ist hier vorgesehen, dass der radiale Kurvenverlauf einer Referenzverbindungskurve zumindest über 50 %, insbesondere zumindest 75 %, insbesondere zumindest 80%, insbesondere zumindest 90%,, insbesondere 100%, einem an einer auf der optischen Hauptachse senkrecht stehenden Ebene der Augenlinse gespiegelten Konturenverlauf der Oberfläche der gegenüberliegenden anderen Seite entspricht. Dadurch wird ein äußerst hoher Symmetriegrad erreicht. Auch diese Ausgestaltung trägt vorteilhaft zur Reduzierung des Linsenvolumens bei zumindest gleichbleibenden optischen Abbildungseigenschaften bei. Ein wesentlicher Vorteil dieser Ausgestaltung ist darin zu sehen, dass durch diese Rotationssymmetrie die Faltungs- und Injektionseigenschaften der Augenlinse, wenn sie als Intraokularlinse ausgebildet ist, verbessert sind.

Darüber hinaus reduziert sich durch die radial gestufte Oberflächenstruktur, insbesondere die Fresnellierung, die Amplitude der Oberflächenmodulation, was unmittelbar zu geringeren Beschleunigungswerten des Werkzeugs, insbesondere eines Drehmeißels, beim Herstellen der Augenlinse beiträgt und somit kürzeren Fertigungszeiten Rechnung trägt.

Vorzugsweise ist vorgesehen, dass ein radialer Konturenverlauf eines flachen Hauptmeridians des torisch brechenden Oberflächenprofils gleich einem Konturenverlauf einer Referenzverbindungskurve, beispielsweise einer Einhüllenden, ist, die auf einer Seite ausgebildete Stufen jeweils, bezogen auf die individuellen axialen Längen der Stufen, auf im Vergleich der Längen der Stufen gleicher axialer Längenverhältnislage schneidet, insbesondere die Stufenspitzen von die gestufte Oberflächenstruktur in einem steilen Hauptmeridian des torisch brechenden Oberflächenprofils bildenden Stufen verbindet. Auch dies ist eine vorteilhafte Ausführung und ermöglicht, die bereits oben genannten Vorteile zu realisieren.

Insbesondere ist diese Einhüllende gleich dem Konturenverlauf in einem ungestuften flachen Hauptmeridian. Bei einer derartigen Ausführung ist somit insbesondere dann auch eine Rotationssymmetrie des Konturenverlaufs auf der Seite, auf welcher das torisch brechende Oberflächenprofil und die gestufe Oberflächenstruktur ausgebildet sind, erzeugt. Die Stufung ist jedoch hier nicht vollständig umlaufend um die Haupotachse ausgebildet, sondern in dem flachen Hauptmeridianen gleich Null.

Insbesondere ist dieser rotationssymmetrische Konturenverlauf gleich dem insbesondere ebenfalls rotationssymmetrischen Konturenverlauf der gegenüberliegenden Seite des optischen Teils, der somit eine Spiegelung an einer senkrecht zur optischen Achse stehenden Ebene des optischen Teils ist.

Besonders vorteilhaft ist in einem weiteren Ausführungsbeispiel, dass ein radialer Konturenverlauf einer Einhüllenden, die Stufenspitzen von die gestufte Oberflächenstruktur in einem flachen Hauptmeridian des torisch brechenden Oberflächenprofils bildenden Stufen verbindet, gleich einem Konturenverlauf einer Einhüllenden ist, die Stufenspitzen von die gestufte Oberflächenstruktur in einem steilen Hauptmeridian des torisch brechenden Oberflächenprofils bildenden Stufen verbindet. Hier kann in Verallgemeinerung auch vorgesehen sein, dass nicht nur die Einhüllende herangezogen wird, die Stufenspitzen verbindet, sondern auch hier wiederum allgemein eine Referenzverbindungskurve zugrunde gelegt wird, die auf einer Seite ausgebildete Stufen jeweils, bezogen auf die individuellen axialen Längen der Stufen, auf im Vergleich der Längen der Stufen gleicher axialer Längenverhältnislage schneidet. Die Längenverhältnislage kann hier auch wiederum zwischen 0% und 100% der Stufenhöhen der einzelnen Stufen liegen. Bei dieser Ausgestaltung ist somit die Ausbildung von Stufen vollständig umlaufend um die Achse A realisiert.

Auch hier lässt sich dann eine Augenlinse, insbesondere eine Intraokularlinse, gestalten, die die bereits oben genannten Vorteile aufweist.

Besonders bevorzugt ist es, dass die genannten radialen Konturenverläufe rotationssymmetrisch um die Hauptachse und somit azimutal umlaufend um die Hauptachse ausgebildet sind.

In einer weiteren vorteilhaften Ausgestaltung ist vorgesehen, dass Stufenspitzen von die gestufte Oberflächenstruktur in einem flachen Hauptmeridian des torisch brechenden Oberflächenprofils bildenden Stufen radial an gleicher Stelle ausgebildet sind wie Stufenspitzen von die gestufte Oberflächenstruktur in einem steilen Hauptmeridian des torisch brechenden Oberflächenprofils bildenden Stufen ausgebildet sind. Dies bedeutet, dass die Stufenspitzen zumindest in den Hauptmeridianen, die insbesondere senkrecht aufeinander stehen, jeweils gleichen Radius aufweisen. Bei einer derartigen Ausgestaltung wird in bevorzugter Weise dann die optische Wirkung des torisch brechenden Oberflächenprofils durch eine unterschiedliche Ausgestaltung der Stufenhöhen der einzelnen Stufen in diesen beiden unterschiedlichen Hauptmeridianen erreicht. Es wird also eine Überlagerung generiert, bei der quasi auf das torisch brechende Oberflächenprofil nach oben erhabene Stufen, insbesondere sägezahnartige Stufen, erzeugt werden, die zwar radial in den Hauptmeridianen auf gleicher radialer Lage ausgebildet sind, jedoch im Hinblick auf ihre Stufenhöhen unterschiedlich gestaltet sind.

Vorzugsweise ist vorgesehen, dass Stufenhöhen von die gestufte Oberflächenstruktur in einem flachen Hauptmeridian des torisch brechenden Oberflächenprofils bildenden Stufen unterschiedlich, insbesondere niedriger, sind, als Stufenhöhen von die gestufte Oberflächenstruktur in einem steilen Hauptmeridian des torisch brechenden Oberflächenprofils bildenden Stufen.

Vorzugsweise ist vorgesehen, dass Stufenhöhen von die gestufte Oberflächenstruktur des torisch brechenden Oberflächenprofils bildenden Stufen nur in einem Hauptmeridian, insbesondere dem flachen Hauptmeridian, gleich Null sind. Daraus lassen sich ganz spezifische Oberflächentopographien für Augenlinsen generieren, die dann wiederum individuelle optische Abbildungseigenschaften zur Korrektur von Sehfehlern ermöglichen.

Vorzugsweise ist vorgesehen, dass die erste Seite des optischen Teils und die zweite Seite des optischen Teils jeweils konvex ausgebildet sind.

In einer alternativen Ausführung ist vorgesehen, dass die erste Seite des optischen Teils und die zweite Seite des optischen Teils jeweils konkav ausgebildet sind. Bei einer derartigen Ausgestaltung ist dann die Mittendicke des optischen Teils nochmals wesentlich reduziert. Die Faltbarkeit und Kleinschnitttauglichkeit ist dann erhöht.

Vorzugsweise ist vorgesehen, dass ein sphärischer Brechkraftanteil der Gesamtbrechkraft der Augenlinse jeweils hälftig auf die erste Seite und die zweite Seite verteilt ist. Es kann jedoch auch vorgesehen sein, dass ein sphärischer Brechkraftanteil der Gesamtbrechkraft der Augenlinse zu 25 % bis 35 % auf eine Seite und restlich auf die andere Seite verteilt ist.

Die Stufen der Oberflächenstruktur werden durch in Umlaufrichtung um die optische Hauptachse umlaufende, radial aneinander angrenzende, ringförmige Zonen, die mit ihren Oberflächen versetzt zueinander ausgebildet sind, gebildet. Bei einer bevorzugten Ausführung der Augenlinse ist die Anzahl der Zonen zwischen 5 und 14, insbesondere zwischen 7 und 14, insbesondere 9 oder 10. Vorzugsweise beträgt eine Fläche einer Zone zwischen 2,7 und 3,9 mm$^2$, insbesondere zwischen 2,7 und 3,1 mm$^2$ oder zwischen 3,3 und 3,6 mm$^2$. Die Stufenhöhe beträgt insbesondere zwischen 20 $\mu$m und 80 $\mu$m.

In einer vorteilhaften Ausführung ist vorgesehen, dass ein radialer Verlauf der Gesamtbrechkraft mit einem diskreten Stufenprofil, welches Minima aufweist, ausgebildet ist und die Stufen der gestuften Oberflächenstruktur radial betrachtet an den Brechkraftstufen ausgebildet sind. Insbesondere bedeutet dies somit, dass an Sprungstellen, an denen sich die Gesamtbrechkraft sprunghaft nach oben oder unten ändert, auch eine Stufenspitze einer Stufe ausgebildet ist.

Bei einer weiteren vorteilhaften Ausgestaltung ist vorgesehen, dass die Stufenhöhen der Stufen mit zunehmendem Radius abnehmen.

Insbesondere ist vorgesehen, dass ein Differenzwert zwischen Stufenhöhen an einem steilen Hauptmeridian des torisch brechenden Oberflächenprofils und Stufenhöhen am flachen Hauptmeridian des torisch brechenden Oberflächenprofils der Stufen mit zunehmendem Radius abnimmt. Somit ist ein Differenzwert einer weitere innen liegenden Stufe und somit auch Zone größer als ein Differenzwert einer radial weiter außen liegenden Zone bzw. Stufe.

Allgemein lässt sich die azimutabhängige Gesamtbrechkraft einer erfindungsgemäßen Augenlinse durch nachfolgende Formel darstellen:

$$F_{ges}\,(\Phi, \rho) = F_{Sphäre}\,(\rho) + F_{Cylinder}\,(\Phi, \rho) + F_{HOA}\,(\Phi, \rho) + F_{EDoF}\,(\Phi, \rho) \qquad (1)$$

mit

$\Phi$ = Azimutwinkel  
$\rho$ = radiale Position auf dem optischen Teil  
$F_{ges}$ = azimutabhängige Gesamtbrechkraft  
$F_{Sphäre}$ = sphärische Brechkraft  
$F_{Cylinder}$ = azimutabhängiger zylindrischer Brechkraftanteil  
$F_{HOA}=$ = azimutabhängiger Brechkraftanteil zur Korrektur von Aberration höherer Ordnung (bspw. Koma, Fünfblattfehler etc.)  
$F_{EDoF}$ = azimutabhängiger Brechkraftanteil zur Beeinflussung der Schärfentiefe

Die Gesamtbrechkraft kann auf beide Seiten des optischen Teils in verschiedensten Verhältnissen aufgeteilt werden, um beispielsweise die Stufenhöhen und die lokalen Krümmungen zu reduzieren. In einer vorteilhaften Ausgestaltung wird eine Gleichverteilung auf beide Seiten durchgeführt.

Darüber hinaus wird eine sagittale Höhe S der Topographie in einem Azimut auf einer Seite des optischen Teils durch die nachstehend genannte Formel 2 bestimmbar:

$$s(r, r_0, Q) = \frac{r^2}{r_0 \left(1 + \sqrt{1 - (1+Q)\frac{r^2}{r_0^{\,2}}}\right)} \qquad (2)$$

r = radiale Position auf Linsenoberfläche

$r_0$ = zentraler Krümmungsradius ($r_0 \rightarrow r_{target}$ = Krümmungsradius der Zielfunktion (einhüllende Oberfläche))

Q bzw.. k = konische Konstante

Eine Gleichung zur Bestimmung des azimutalen Scheitelkrümmungsradius beschreibt den radialen Verlauf einer konischen Asphäre. Diese kann durch Addition von Polynomkoeffizienten verallgemeinert oder durch andere Beschreibungsarten, beispielsweise durch Zernike-Reihen, Polynome, Splines oder Bezierkurven oder stückweise definierte Funktionen ersetzt werden. Durch die Variation der Asphärizität, beispielsweise durch Variation der konischen Konstante k, kann der azimutabhängige radiale Brechkraftverlauf beeinflusst werden. Dies erfolgt vorteilhafterweise, um Abbildungsfehler durch sphärische Aberration zu kompensieren. Der in oben genannter Formel bestimmte Wert für die sagittale Höhe und somit die Höhe in Richtung der Achse A betrachtet, kann an jedem beliebigen Ort der Oberfläche einer Seite des optischen Teils berechnet werden. Die Berechnung dieser Sagittalhöhe in jedem Azimut führt dann direkt zur dreidimensionalen Topographie der Seitenoberfläche und somit zur Darstellung der Daten für einen Fertigungsprozess. Die Kleinschnitttauglichkeit wird insbesondere durch Reduktion des Linsenvolumens mittels geometrieabhängiger Fresnellierung erreicht. Dabei ist bei der Fresnellierung vorgesehen, dass der mit zunehmender Radialkoordinate anwachsende Defokus durch die Verschiebung der Krümmungsmittelpunkte in den jeweiligen Zonen, insbesondere Fresnelzonen, durch eine Anpassung der Brechkräfte in den jeweiligen Fresnelzonen kompensiert ist. Die topographieabhängige Fresnellierung ermöglicht, die einhüllende Topographie des optischen Teils in vorteilhafter Weise hinsichtlich Symmetrie und Injektionseigenschaften zu optimieren. Durch eine geeignete azimutale Variation der Stufenhöhe und somit auch einer Fresnelzahntiefe kann eine vorteilhafte Rotationssymmetrie der Einhüllenden in dieser Kontur erreicht werden. Derartige Geometrien erleichtern die Injizierbarkeit der als Intraokularlinse ausgebildeten Augenlinse durch kleine Schnitte im Auge wesentlich.

Bei einer Gleichverteilung der Sphäre und der Fresnellierung des steilen Hauptmeridians entsprechend dem flachen Hauptmeridian ergibt sich auch bei hohen Zylinderwerten der Augenlinse eine Ausgestaltung, welche eine rotationssymmetrische einhüllende Form aufweist und symmetrisch hinsichtlich des Krümmungsverlaufs und der Mittendicke zum Augenlinsenäquator und somit zu einer Symmetrieebene beziehungsweise Hauptebene ist.

Die in der oben genannten Formel angegebene azimutabhängige zylindrische Brechkraft kann auch sehr klein sein und vorzugsweise < 0,01 Dioptrien. Vorzugsweise ist sie jedoch größer.

Es ist auch möglich, dass die Oberflächentopographie einer der beiden Seiten, auf der dann beispielsweise keine gestufte Oberflächenstruktur und kein torisch brechendes Oberflächenprofil ausgebildet sind, unabhängig von der Brechkraft der Augenlinse an die anatomischen Gegebenheiten des Kapselsacks angepasst werden, in welchen die Intraokularlinse eingesetzt wird. Die Augenlinse kann auch eine multifokale Linse sein und kann in dem Zusammenhang eine Diffraktionslinse sein. Sie kann eine Mehrzahl von in radialer Richtung aneinander angrenzend ausgebildeten, zumindest teilweise um die optische Achse ringförmig umlaufende Zonen umfassen, die insbesondere jeweils zumindest eine Hauptunterzone und eine Phasenunterzone aufweisen. Mittels einer derartigen Unterzonengestaltung einer Gesamtzone werden Ausgestaltungen erreicht, bei denen unerwünschte Interferenzeigenschaften kompensiert werden können, was insbesondere durch die Phasenunterzone erreicht wird.

Weitere Merkmale der Erfindung ergeben sich aus den Ansprüchen, den Figuren und der Figurenbeschreibung. Die vorstehend in der Beschreibung genannten Merkmale und Merkmalskombinationen, sowie die nachfolgend in der Figurenbeschreibung genannten und/oder in den Figuren alleine gezeigten Merkmale und Merkmalskombinationen sind nicht nur in der jeweils angegebenen Kombination, sondern auch in anderen Kombinationen oder in Alleinstellung verwendbar, ohne den Rahmen der Erfindung zu verlassen. Es sind somit auch Ausführungen von der Erfindung als umfasst und offenbart anzusehen, die in den Figuren nicht explizit gezeigt und erläutert sind, jedoch durch separierte Merkmalskombinationen aus den erläuterten Ausführungen hervorgehen und erzeugbar sind.

Die in den Unterlagen angegebenen konkreten Werte von Parametern und Angaben zu Verhältnissen von Parametern bzw. Parameterwerten zur Definition von Ausführungsbeispielen der Augenlinse sind auch im Rahmen von Abweichungen, beispielsweise aufgrund von Messfehlern, Systemfehlern, DIN-Toleranzen etc. als vom Rahmen der Erfindung mit umfasst anzusehen, wodurch auch Erläuterungen, die sich auf im Wesentlichen entsprechende Werte und Angaben beziehen darunter zu verstehen sind.

Kurze Beschreibung der Zeichnungen

Ausführungsbeispiele der Erfindung werden nachfolgend anhand schematischer Zeichnungen näher erläutert. Es zeigen:

Fig. 1a       eine perspektivische Darstellung eines ersten Ausführungsbeispiels einer erfindungsgemäßen Augenlinse;

Fig. 1b       eine perspektivische Darstellung eines weiteren Ausführungsbeispiels einer erfindungsgemäßen Augenlinse;

Fig. 2a bis 2d      eine Darstellung von Verläufen der Gesamtbrechkraft, der zylindrischen Brechkraft und der sphärischen Brechkraft sowie des Radius in Abhängigkeit vom Azimut für ein Ausführungsbeispiel einer erfindungsgemäßen Augenlinse;

Fig. 2e      ein Diagramm, in dem die sagittale Höhe einer torischen Intraokularlinse gemäß dem Stand der Technik in zwei senkrecht aufeinander stehenden Hauptmeridianen dargestellt ist;

Fig. 2f      ein Diagramm, in dem die sagittale Höhe des Ausführungsbeispiels gemäß Fig. 2a bis 2d gezeigt ist;

Fig. 2g      ein Diagramm, in dem die sagittale Höhendifferenz zwischen den zwei Hauptmeridianen, wie sie in Fig. 2e dargestellt ist, gezeigt ist;

Fig.2h      ein Diagramm, in dem die sagittale Höhendifferenz des Ausführungsbeispiels der erfindungsgemäßen Augenlinse in Fig. 2f gezeigt ist;

Fig. 2i      ein Diagramm, in dem die Breiten des tangentialen Fokus und des sagittalen Fokus des Ausführungsbeispiels der erfindungsgemäßen Augenlinse gezeigt sind;

Fig. 2j      eine dreidimensionale Darstellung einer Oberflächentopographie der gemäß den Diagrammen in Fig. 2e und 2g aus dem Stand der Technik bekannten Intraokularlinse;

Fig. 2k      eine Darstellung einer dreidimensionalen Oberflächentopographie einer Seite eines optischen Teils des Ausführungsbeispiels der erfindungsgemäßen Augenlinse, wie sie in Fig. 2a bis 2d und Fig. 2f und 2h sowie 2i gezeigt ist;

Fig. 2l      eine Darstellung des Verlaufs einer Rückseite sowie einer Vorderseite der aus dem Stand der Technik bekannten Intraokularlinse in einem Diagramm, in dem die sagittale Höhe in Abhängigkeit vom Radius dargestellt ist;

Fig. 2m      eine Darstellung des Verlaufs einer Rückseite und einer Vorderseite des Ausführungsbeispiels der erfindungsgemäßen Augenlinse, wie sie in oben genannten Diagrammen dargestellt ist, in einem Diagramm, in dem die sagittale Höhe in Abhängigkeit vom Radius gezeigt ist;

Fig. 3a bis 3d      eine Darstellung von Diagrammen, in denen die Gesamtbrechkraft, die zylindrische Brechkraft und die sphärische Brechkraft eines weiteren Ausführungsbeispiels einer erfindungsgemäßen Augenlinse, als auch der Verlauf des Radius der Linse in Abhängigkeit vom Azimut gezeigt sind;

Fig. 3e      ein Diagramm, in dem die sagittale Höhe an zwei senkrecht aufeinander stehenden Hauptmeridianen in Abhängigkeit vom Radius einer aus dem Stand der Technik bekannten Augenlinse dargestellt ist;

Fig. 3f      eine Darstellung eines Diagramms, in dem die sagittale Höhe in Abhängigkeit vom Radius für das erfindungsgemäße Ausführungsbeispiel einer Augenlinse gemäß Fig. 3a bis 3d an zwei unterschiedlichen, senkrecht aufeinander stehenden Hauptmeridianen gezeigt ist;

Fig. 3g      ein Diagramm, in dem die sagittale Höhendifferenz zwischen den beiden in Fig. 3e gezeigten Konturenverläufen in den Hauptmeridianen dargestellt ist;

Fig. 3h      ein Diagramm, in dem die sagittale Höhendifferenz zwischen den zwei senkrecht aufeinander stehenden Hauptmeridianen, wie sie in dem erfindungsgemäßen Ausführungsbeispiel einer Augenlinse in Fig. 3f gezeigt sind, dargestellt ist;

Fig. 3i      ein Diagramm, in dem die Breite des tangentialen Fokus und des sagittalen Fokus des Ausführungsbeispiels gemäß Fig. 3a bis 3d und 3f und 3h gezeigt ist;

Fig. 3j      ein Diagramm, in dem der Konturenverlauf der Rückseite und der Vorderseite einer Intraokularlinse aus dem Stand der Technik gezeigt ist;

Fig. 3k      ein Diagramm, in dem die sagittale Höhe in Abhängigkeit vom Radius für eine Rückseite und eine Vorderseite des optischen Teils der erfindungsgemäßen beispielhaften Augenlinse, wie sie in Fig. 3a bis 3d und Fig. 3f sowie Fig. 3h und 3i gezeigt ist, dargestellt sind;

Fig. 3l      ein Diagramm, in dem die sagittale Höhe in Abhängigkeit vom Radius für ein weiteres Ausführungsbeispiel einer erfindungsgemäßen Augenlinse dargestellt ist, bei welcher im Unterschied zur Darstellung in Fig. 3k sowohl die Vorderseite als auch die Rückseite konkav gekrümmt sind;

Fig. 4      ein Diagramm, in welchem ein Zusammenhang zwischen einer Anzahl der eine radial gestufte Oberflächenstruktur erzeugenden ringförmigen optischen Zonen, einer Flächengröße und der Stufenhöhe gezeigt ist;

Fig. 5a      eine Tabelle, in welcher Werte für den maximalen Radius, den Radius an einem flachen Hauptmeridian und an einem steilen Hauptmeridian sowie die Krümmung in dem flachen und dem steilen Hauptmeridian, die Krümmungsdifferenz, die Stufenhöhe im flachen und im steilen Hauptmeridian die Stufenhöhe für eine beispielhafte Anzahl von Zonen einer Augenlinse dargestellt sind;

Fig. 5b      eine dreidimensionale Darstellung einer Oberflächentopographie der Vorderseite gemäß der Linse in dem Diagramm in Fig. 5a;

Fig. 6a      eine Tabelle, in welcher Werte für den maximalen Radius, den Radius an einem flachen Hauptmeridian und an einem steilen Hauptmeridian sowie die Krümmung in dem flachen und dem steilen Hauptmeridian, die Krümmungsdifferenz, die Stufenhöhe im flachen und im steilen Hauptmeridian die Stufenhöhe für eine beispielhafte Anzahl von Zonen einer Augenlinse dargestellt sind;

Fig. 6b      eine dreidimensionale Darstellung einer Oberflächentopographie der Vorderseite gemäß der Linse in dem Diagramm in Fig. 6a;

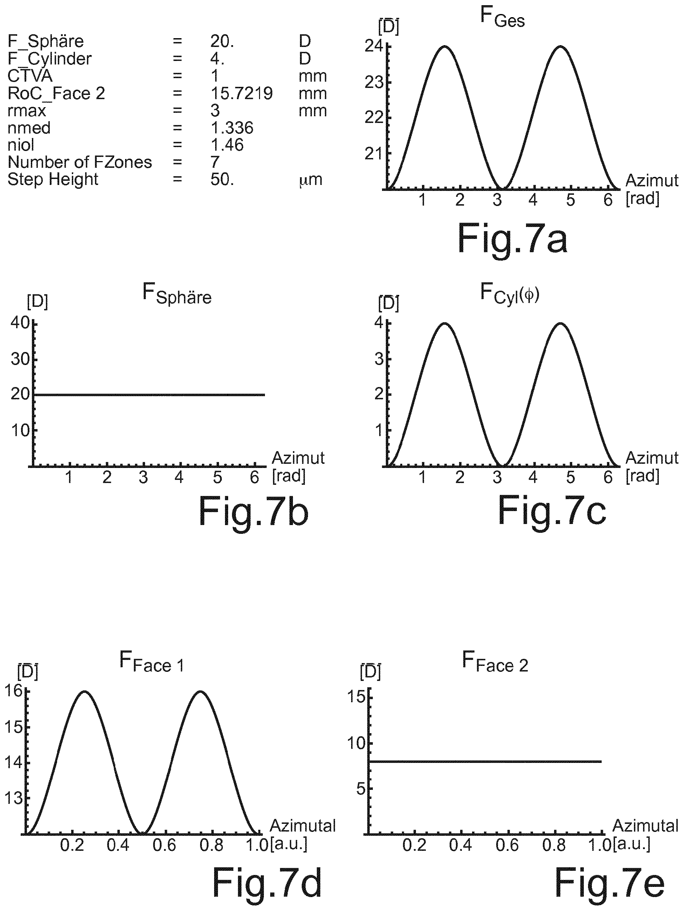

Fig. 7a bis 7j      eine Darstellung von Diagrammen, in denen die Gesamtbrechkraft, die sphärische Brechkraft und die zylindrische Brechkraft eines weiteren Ausführungsbeispiels einer erfindungsgemäßen Augenlinse, als auch der Verlauf der Brechkraft auf einer ersten Seite des optischen Teils und auf einer zweiten Seite des optischen Teils in Abhängigkeit vom Azimut dargestellt sind, und die Gesamtbrechkraft, der Radius, insbesondere der Scheitelradius, die Krümmung, insbesondere die Scheitelkrümmung, die konische Konstante und die sagittale Höhe der Hauptmeridiane in Abhängigkeit vom normierten Azimutwinkel dargestellt sind ;

Fig. 7k      eine Tabelle, in welcher Werte für den maximalen Radius, den Radius an einem flachen Hauptmeridian und an einem steilen Hauptmeridian sowie die Stufenhöhe für eine beispielhafte Anzahl von Zonen der Augenlinse gemäß Fig. 7a bis 7j dargestellt sind;

Fig. 7l      ein Diagramm, in welchem die sagitalle Höhe der Vorderseite mit den Konturenverläufen im flachen und im steilen Hauptmeridian, und der Konturenverlauf der Rückseite des optischen Teils der Augenlinse gemäß Fig. 5a bis 5k dargerstellt sind;

Fig. 7m      eine dreidimensionale Darstellung einer Oberflächentopographie der Vorderseite der Linse gemäß den Diagrammen in Fig. 7a und 7l;

Fig. 8a      eine Tabelle, in welcher Werte für den maximalen Radius, den Radius an einem flachen Hauptmeridian und an einem steilen Hauptmeridian sowie die Stufenhöhe für eine beispielhafte Anzahl von Zonen einer Augenlinse dargestellt sind, die im Vergleich zu Fig. 7a bis 7m eine zylindrische Brechkraft von 8dpt und einen Radius der Rückseite von 14,4027 mm aufweist;

Fig. 8b      eine dreidimensionale Darstellung einer Oberflächentopographie der Vorderseite gemäß der Linse in dem Diagramm in Fig. 8a;

Fig. 9      eine Tabelle, in welcher Werte für den maximalen Radius, den Radius an einem flachen Hauptmeridian und an einem steilen Hauptmeridian sowie die Krümmung in dem flachen und dem steilen Hauptmeridian, die Krümmungsdifferenz, die Stufenhöhe im steilen Hauptmeridian für eine beispielhafte Anzahl von Zonen einer Augenlinse dargestellt sind;

Fig. 10    ein Diagramm, in welchem ein Zusammenhang zwischen einer Anzahl der eine radial gestufte Oberflächenstruktur erzeugenden ringförmigen optischen Zonen, einer zylindrischen Brechkraft und einer sphärischen Brechkraft für Ausführungsbeispiele von erfindungsgemäßen Augenlinsen gezeigt ist; und

Fig. 11    eine Tabelle, in welcher für unterschiedliche sphärische Brechkräfte $F_{sph}$, unterschiedliche zylindrische Brechkräfte $F_{cyl}$, unterschiedliche Zonenzahl N Querschnittflächen (A_cross) des optischen Teils für Ausführungsbeispiele erfindungsgemäßer Augenlinsen in zwei senkrecht aufeinander stehenden Querschnittebenen angegeben sind.

Bevorzugte Ausführung der Erfindung

In den Figuren werden gleiche oder funktionsgleiche Elemente mit den gleichen Bezugszeichen versehen.

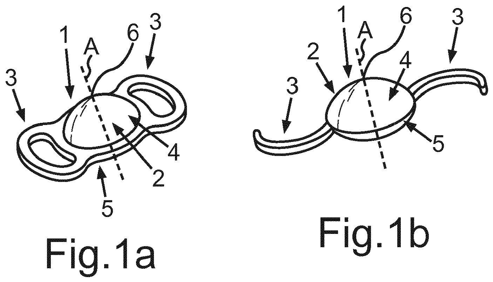

In Fig. 1a ist in einer perspektivischen Darstellung ein erstes Ausführungsbeispiel einer Augenlinse 1 gezeigt, die eine Intraokularlinse ist. Die Augenlinse 1 umfasst einen optischen Teil 2 und daran anschließend eine Haptik 3. Die Augenlinse 1 ist faltbar und über einen Kleinschnitt in ein Auge einführbar. Der optische Teil 2, der wesentlich für die optische Abbildungseigenschaft der Augenlinse 1 ist, umfasst eine optische Hauptachse A. Der optische Teil 2 weist darüber hinaus in Richtung dieser optischen Hauptachse A betrachtet eine erste optische Fläche bzw. Seite 4, die eine Vorderseite sein kann, und gegenüberliegend eine zweite optische Fläche bzw. Seite 5, die eine Rückseite sein kann, auf. Die beispielhafte Vorderseite 4 ist im implantierten Zustand der Augenlinse 1 im Auge der Hornhaut zugewandt, wohingegen die Rückseite dieser Hornhaut abgewandt ist.

In Fig. 1b ist in einer perspektivischen Darstellung ein weiteres Ausführungsbeispiel einer als Intraokularlinse ausgebildeten Augenlinse 1 gezeigt. Sie unterscheidet sich von der Ausführung in Fig. 1a durch die unterschiedliche Haptik 3. Mittels der Haptik 3 ist die Augenlinse 1 im Auge gehalten.

Grundsätzlich können auch anderweitig geformte und ausgestaltete Haptiken 3 vorgesehen sein.

Die Augenlinsen 1 gemäß Fig. 1a und 1b weisen in ihren optischen Teilen 2 an der einen Seite 4 und der gegenüberliegenden Seite 5 Scheitel 6 auf.

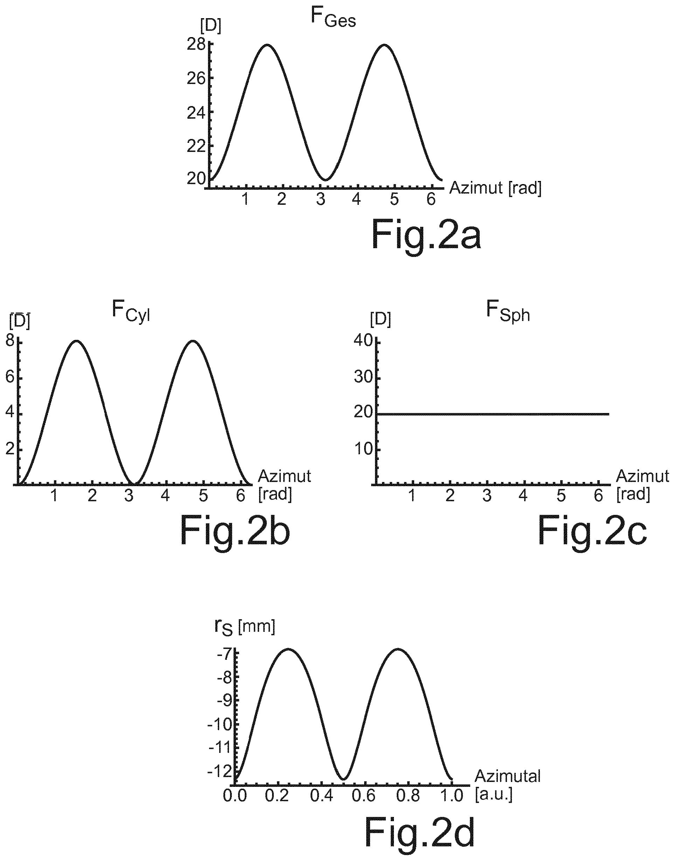

In Fig. 2a ist ein Diagramm gezeigt, in welchem die Gesamtbrechkraft $F_{Ges}$ eines Ausführungsbeispiels einer erfindungsgemäßen Augenlinse 1 in Abhängigkeit vom Azimut bei einem vollständigen Umlauf um die Achse A gezeigt ist. Der wellenförmige Verlauf mit Minima und Maxima ist zu erkennen, wobei dazu azimutal die Gesamtbrechkraft zwischen den Werten 20 Dioptrien und 28 Dioptrien variiert.

In Fig. 2b ist ein Diagramm gezeigt, in welchem der Verlauf der azimutalen zylindrischen Brechkraft, die durch ein torisch brechendes Oberflächenprofil zur Gesamtbrechkraft beigetragen wird, dargestellt ist. Diese zylindrische Brechkraft $F_{Cyl}$ variiert hier zwischen 0 und 8 Dioptrien.

Darüber hinaus ist in Fig. 2c ein Diagramm gezeigt, in welchem die sphärische Brechkraft $F_{Sphäre}$ in Abhängigkeit vom Azimut bei einem vollständigen Umlauf um die Achse A dargestellt ist. Dieser ist im Ausführungsbeispiel konstant und beträgt beispielhaft den Wert 20.

Darüber hinaus ist in dem Diagramm gemäß Fig. 2d der Radius, insbesondere der Scheitelradius, in Abhängigkeit vom Azimutwinkel dargestellt, der hier im Vergleich zu den Diagrammen gemäß Fig. 2a bis 2c nicht in der Einheit [Rad] angegeben ist, sondern in normierter Form dargestellt ist. Die wellenförmige Variation dieses Radius $r_S$ ist gezeigt.

Sowohl der Verlauf der zylindrischen Brechkraft als auch der Wert der sphärischen Brechkraft als auch der Verlauf des Krümmungsradius ist beispielhaft.

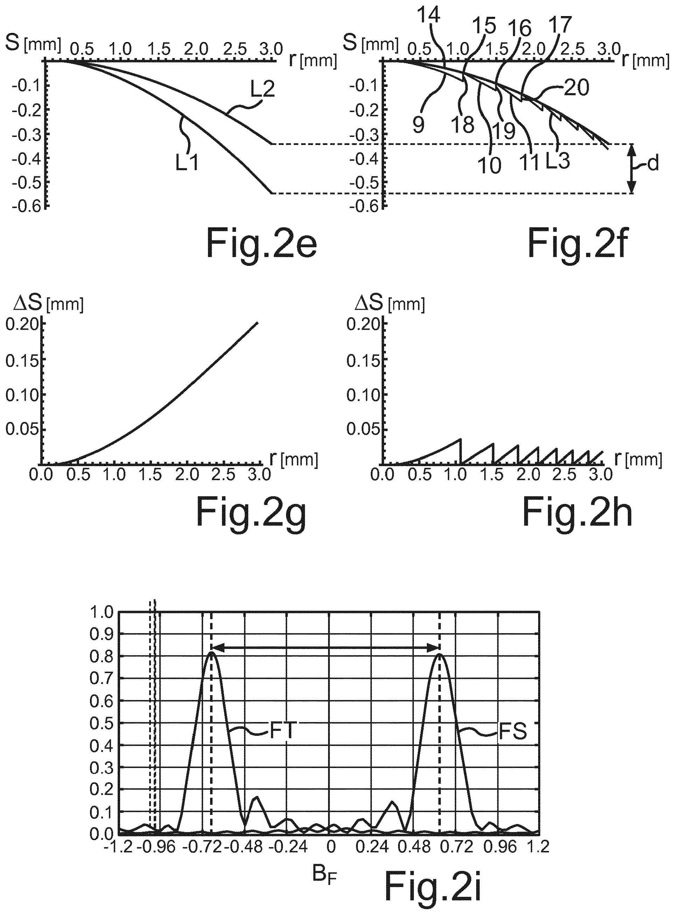

In Fig. 2e ist ein Diagramm gezeigt, in welchem die sagittale Höhe S in Abhängigkeit vom Radius r des optischen Teils 2 bei einer torischen Intraokularlinse aus dem Stand der Technik dargestellt ist. In dem Zusammenhang zeigt die Kurve L1 den Verlauf am steilen Hauptmeridian, wohingegen die Kurve L2 den Verlauf am dazu um 90° versetzten flachen Hauptmeridian zeigt.

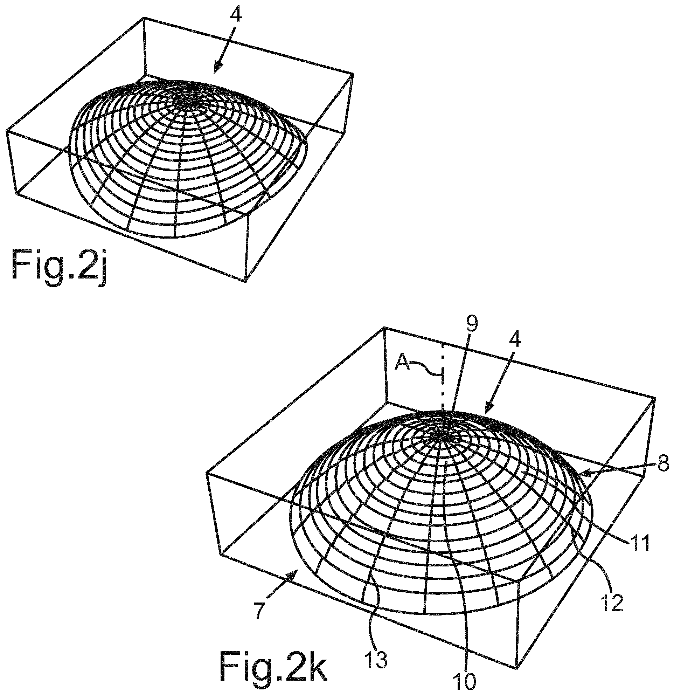

Im Vergleich dazu ist in Fig. 2f ein Diagramm gezeigt, in welchem die sagittale Höhe in Abhängigkeit vom Radius des Ausführungsbeispiels einer erfindungsgemäßen Augenlinse 1 gezeigt ist. In dem Zusammenhang ist zu erwähnen, dass eine Seite 4 oder eine Seite 5 oder beide Seiten 4 und 5 entsprechend mit einer Oberflächentopographie gestaltet sind, die ein torisch brechendes Oberflächenprofil 7 (Fig. 2k) und zusätzlich zum torisch brechenden Oberflächenprofil 7 eine in radialer Richtung des optischen Teils 2 sägezahnartig gestufte Oberflächenstruktur 8 aufweist, wobei die sägezahnartig gestufte Oberflächenstruktur 8 ebenfalls auf zumindest einer Seite 4, 5 ausgebildet ist.

Bei dem Ausführungsbeispiel gemäß Fig. 2k, in welchem dreidimensional eine Oberflächentopographie des Ausführungsbeispiels dargestellt ist, ist das torisch brechende Oberflächenprofil 7 und die radial sägezahnartig gestufte Oberflächenstruktur 8 auf einer Seite, beispielsweise der Seite 4, überlagert ausgebildet.

Im Ausführungsbeispiel ist vorgesehen, dass die gestufte Oberflächenstruktur 8 eine Fresnellierung ist und eine Mehrzahl von ringförmig umlaufenden Zonen 9, 10, 11 umfasst, wobei hier zu erwähnen ist, dass der Übersichtlichkeit dienend nur einige der in radialer Richtung direkt aneinander anschließenden ringförmigen Zonen mit einem entsprechenden Bezugzeichen versehen sind. Diese Zonen 9 bis 11, von denen auch die innerste, eine Kreisfläche darstellende, Zone 9 als ringförmige Zone bezeichnet wird, und an ihren radialen inneren und äußeren Grenzen an benachbarte Zonen angrenzen, wobei zwischen zwei Zonen jeweils eine Stufe ausgebildet ist. Dies bedeutet, dass die angrenzenden Ränder der benachbarten Zonen in Richtung der Achse A um eine insbesondere in axialer Richtung orientierte Stufenhöhe versetzt zueinander ausgebildet sind.

In der Darstellung in Fig. 2f ist eine Kurve L3 gezeigt, die den gestuften Verlauf der auf der Seite 4 bei einem steilen Hauptmeridian 13, der im Vergleich zu einem flachen Hauptmeridian 12 des torisch brechenden Oberflächenprofils 7 einen kleineren Radius aufweist, entsprechende Stufen zwischen den Zonen 9 bis 11 zeigt.

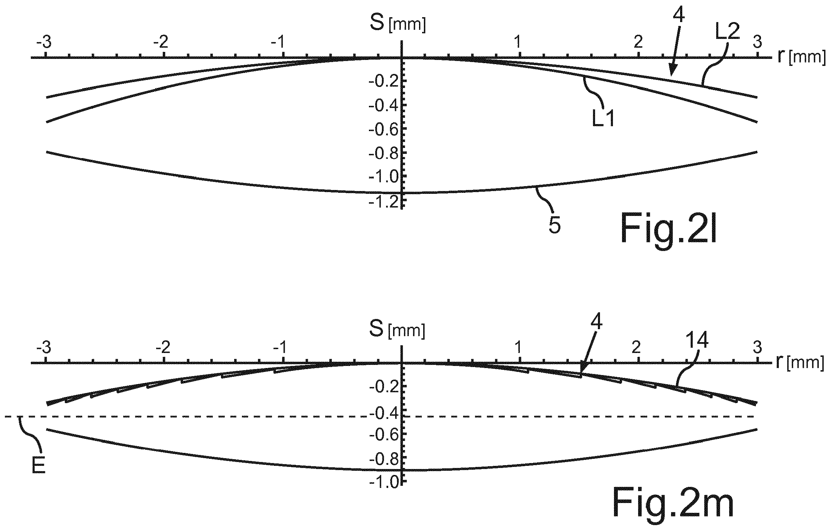

Darüber hinaus ist zu erkennen, dass ein radialer Konturenverlauf einer Einhüllenden 14, die die auf einer Seite 4 ausgebildeten Stufenspitzen 15, 16 und 17 von die gestufte Oberflächenstruktur 8 bildenden Stufen 9 bis 11 verbindet, zumindest abschnittsweise, insbesondere vollständig, einem an einer auf der optischen Hauptachse A senkrecht stehenden Geometrieebene E (Fig. 2m) des optischen Teils 2 der Augenlinse 1 gespiegelten Konturenverlauf der Oberfläche der gegenüberliegenden anderen Seite 5 entspricht. Dies bedeutet, dass gemäß der Darstellung in Fig. 2m, in der die sagittale Höhe S in Abhängigkeit vom Radius r des Ausführungsbeispiels der erfindungsgemäßen Augenlinse 1 dargestellt ist, die Seite 5, insbesondere eine Rückseite, darstellt, die der an der Ebene E gespiegelten Einhüllenden 14, die die Stufenspitzen 15 bis 17, die stellvertretend für alle anderen Stufenspitzen der weiteren Stufen mit einem Bezugzeichen versehen sind, verbindet, entspricht.

In dem Ausführungsbeispiel gemäß Fig. 2f ist auch vorgesehen, dass der radiale Konturenverlauf eines flachen Hauptmeridians 12 des torisch brechenden Oberflächenprofils gleich dem Konturenverlauf der Einhüllenden 14 ist. Insbesondere ist der Konturenverlauf der Seite 4 rotationssymmetrisch um die Achse A.

Anstelle der Einhüllenden 14 kann jedoch auch eine andere Referenzverbindungskurve zugrunde gelegt werden, die beispielsweise nicht die Stufenspitzen, sondern die Stufenböden der Zonen 9 bis 11 verbindet. Ebenso kann eine Referenzverbindungskurve zugrunde gelegt werden, die jeweils abhängig von einem prozentualen Längenverhältnis und somit von einer axialen Längenverhältnislage jede einzelnen Stufenhöhe von Stufen 18, 19 und 20, die wiederum stellvertretend für alle anderen Stufen mit Bezugzeichen versehen sind, diese schneidet. So kann vorgesehen sein, dass die Referenzverbindungskurve alle Stufen in ihrer Stufenhöhe bei 50 % der gesamten Stufenhöhe jeweils schneidet.

Durch diese einzeln als auch in Kombination realisierbaren vorteilhaften Ausführungen wird der spezifischen radialen sägezahnartigen Stufung, der Gestaltung der Stufen 18 bis 20 und/oder der Stufenspitzen 15 bis 17 derart, dass sie auf einer Referenzverbindungskurve liegen, die spiegelsymmetrisch zu einer gegenüberliegenden Seite 5 des optischen Teils sind und/oder bei welchen der flache Meridian im Verlauf der Einhüllenden 14, die die Stufenspitzen 15 bis 17 verbindet, entspricht, kann eine wesentliche Reduzierung des Volumens des optischen Teils 2 erreicht werden. Beispielhaft ist in Fig. 2e und 2f gezeigt, um wie viel sich eine Reduktion der Mittendicke des optischen Teils 2 erreichen lässt, was in Fig. 2f durch die Dicke d angedeutet ist, die beispielhaft zwischen 0,15 und 0,25, insbesondere im Wesentlichen 0,2 mm beträgt.

In Fig. 2g ist in einem Diagramm die Differenz der sagittalen Höhe bzw. die Sagittalhöhendifferenz ΔS in Abhängigkeit von dem Radius der beiden Kurven L1 und L2 in Fig. 2e gezeigt. Es ist somit ein Verlauf gezeigt, wie er bei Linsen aus dem Stand der Technik auftritt. Es ist zu erkennen, dass mit zunehmendem Radius r diese Sagittalhöhendistanz zunimmt, insbesondere stark zunimmt.

Demgegenüber ist in Fig. 2h diese Sagittalhöhendifferenz ΔS für ein Ausführungsbeispiel der Erfindung, wie es zu Fig. 2f erläutert wurde, gezeigt. Es ist hier zu erkennen, dass mit zunehmendem Radius diese Sagittalhöhendifferenz ΔS kleiner wird. Insbesondere ist in dem Zusammenhang vorteilhaft, wenn ein Differenzwert zwischen der Stufenhöhe am steilen Hauptmeridian 13 und der Stufenhöhe am flachen Hauptmeridian 12 der Stufen mit zunehmendem Radius r abnimmt. Bei der Darstellung in Fig. 2a ist zu erkennen, dass die maximale Sagittalhöhendifferenz bei etwa 0,035 mm liegt und dann radial abnimmt.

In Fig. 2i ist ein Diagramm gezeigt, in welchem die Breiten $B_F$ von einem tangentialen Fokus FT und einem sagittalen FS dargestellt sind. Es ist hier ein Astigmatismus von etwa 6 Dioptrien zugrunde gelegt, der dem Abstand der Maxima des tangentialen Fokus und des sagittalen Fokus entspricht.

In Fig. 2j ist im Vergleich zur Darstellung in Fig. 2k eine dreidimensionale Darstellung einer Oberflächentopographie einer Seite eines optischen Teils einer aus dem Stand der Technik bekannten torischen Intraokularlinse gezeigt. Die unterschiedlichen Radien sind in dem Zusammenhang deutlich zu erkennen.

Demgegenüber ist in Fig. 2k in azimutaler Richtung bei allen Zonen ein konstanter Radius ausgebildet, und es wird die Überlagerung des torisch brechenden Oberflächenprofils 7 mit der sägezahnartig gestuften Oberflächenstruktur 8 dahingehend erreicht, dass die Stufenhöhen in azimutaler Richtung variieren und insbesondere an den Meridianen 12 und 13 maximal unterschiedlich sind.

In Fig. 2l ist ein Diagramm gezeigt, bei dem die sagittale Höhe S in Abhängigkeit vom Radius r für die bekannte Linse gemäß Fig. 2j gezeigt ist. Es ist hier auch die Seite 5 gezeigt, wobei die Seite 4 mit dem flacheren Verlauf L2 im flacheren Hauptmeridian und L1 im steileren Hauptmeridian gezeigt ist. Eine symmetrische Ausgestaltung zu einer Ebene E ist in Fig. 2l völlig fernliegend.

Darüber hinaus wird durch die Vergleiche von Fig. 2l und einem Ausführungsbeispiel einer erfindungsgemäßen Augenlinse gemäß Fig. 2m auch wieder die Reduzierung der Mittendicke deutlich. Auch ist die rotationssymmetrische Ausgestaltung der Einhüllenden 14 zu erkennen, die somit auch eine rotationssymmetrische Ausgestaltung der säge-zahnartig gestuften Oberflächenstruktur 7 verdeutlicht.

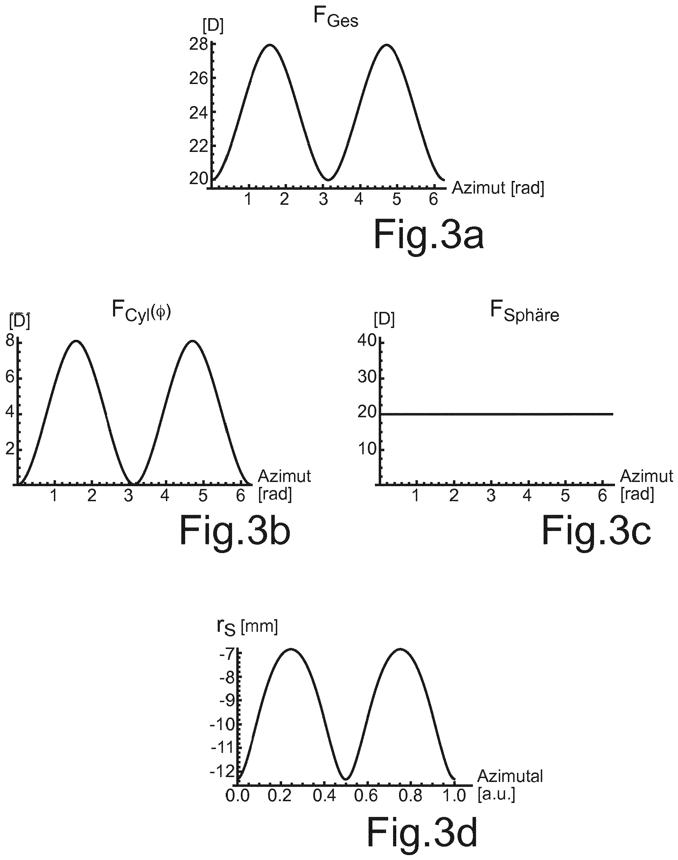

In Fig. 3a ist ein Diagramm gezeigt, bei dem die Gesamtbrechkraft $F_{Ges}$ in Abhängigkeit vom Azimut für einen vollständigen Umlauf um die Achse A für ein weiteres Ausführungsbeispiel einer erfindungsgemäßen Augenlinse 1 gezeigt ist.

Darüber hinaus ist in Fig. 3b ein Diagramm gezeigt, in welchem die zylindrische Brechkraft $F_{Cyl}$ in Abhängigkeit vom Azimut gezeigt ist. Dadurch ist auch der Anteil des torisch brechenden Oberflächenprofils 7 an der Gesamtbrechkraft verdeutlicht. Darüber hinaus ist in Fig. 3c ein Diagramm gezeigt, in welchem die sphärische Brechkraft $F_{Sphäre}$ in Abhängigkeit vom Azimut dargestellt ist und beispielhaft konstant, insbesondere den Wert 0 beträgt.

In dem Diagramm gemäß Fig. 3b ist wiederum der Radius, insbesondere der Scheitelradius $r_S$ in Abhängigkeit vom normierten Azimutwinkel gezeigt.

Bei dem Ausführungsbeispiel in Fig. 2a bis 2m ist vorgesehen, dass Brechkräfte der Sphäre zu gleichen Teilen auf die Seite 4 und die Seite 5 der Augenlinse 1 verteilt sind. Daraus ergibt sich trotz des hohen Zylinderwertes eine Augenlinse, welche eine rotationssymmetrische Einhüllende 14 besitzt und darüber hinaus symmetrisch hinsichtlich des Krümmungsverlaufs und der Mittendicke zum Linsenäquator und somit zur Ebene E ist. Durch die spezifische Fresnellierung ist die Einhüllende 14 des steilen Meridians 13 exakt an den flachen Meridian 12 angepasst, wie dies in Fig. 2f und 2m gezeigt ist.

Beim Ausführungsbeispiel in Fig. 3a ff. ist insbesondere vorgesehen, dass die Verteilung der Brechkräfte auf die Seite 4 und auf die Seite 5 nicht hälftig ist, sondern, dass beispielsweise Brechkräfte der Sphäre 25 % bis 35 % auf eine Seite 4 oder 5 und der restliche Anteil auf der anderen Seite 5 beziehungsweise 4 verteilt ist. Dadurch ist es ermöglicht, den Krümmungsradius der Seite, die den kleineren Brechkraftanteil aufweist, um zumindest 80%, insbesondere 90 % zu erhöhen. Beispielsweise kann vorgesehen sein, dass die Verteilung der Brechkräfte der Sphäre auf der Vorderseite 6,2 Dioptrien und auf der Rückseite 13,8 Dioptrien beträgt, wenn die gesamte sphärische Brechkraft 20 Dioptrien beträgt, wie dies in Fig. 3c verdeutlicht ist.

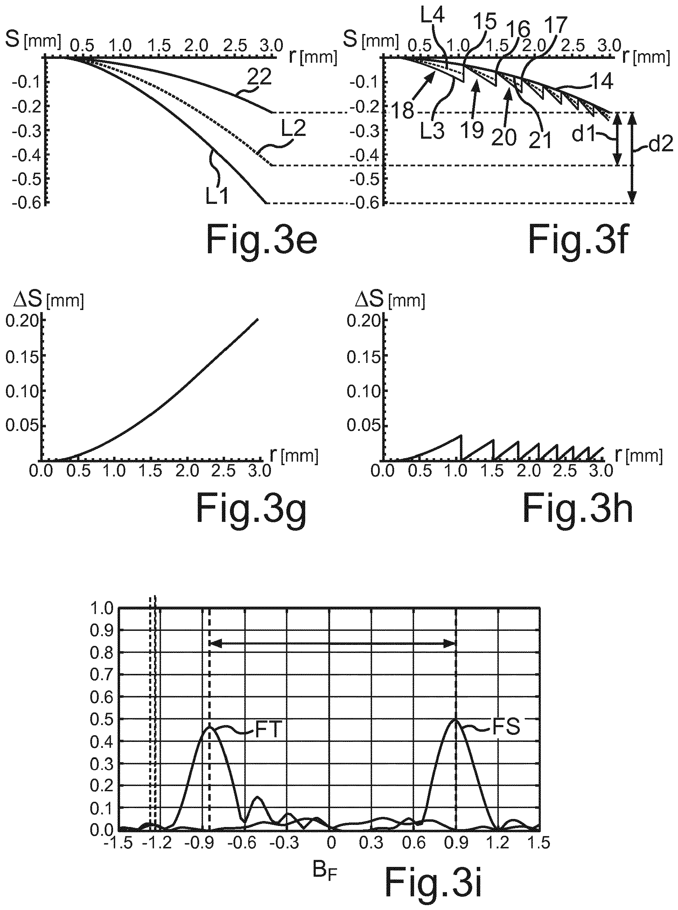

Die Fresnellierung anhand einer rotationssymmetrischen Zielfunktion erzeugt unabhängig vom hohen Zylinderwert der Augenlinse 1, welche eine rotationssymmetrische Einhüllende 21 gemäß Fig. 3f aufweist, eine deutlich reduzierte Mittendicke gegenüber dem Stand der Technik, wie dies im Vergleich von Fig. 3e und Fig. 3f gezeigt ist.

Bei dieser Ausgestaltung ist vorgesehen, dass durch die Kurve L3 das gestufte Profil beziehungsweise der gestufte Konturenverlauf am steilen Hauptmeridian 13 verdeutlicht ist, wohingegen durch die Kurve L4 der Konturenverlauf vom flachen Meridian 12 verdeutlicht ist. Wie aus der Darstellung in Fig. 3f, die die sagittale Höhe S in Abhängigkeit vom Radius r darstellt, zu erkennen ist, sind die Zonen 18 bis 20 in radialer Richtung im steilen Hauptmeridian 13 und im flachen Hauptmeridian 12 gleich groß. Darüber hinaus weisen auch die Stufenspitzen 15, 16 und 17 die beiden Hauptmeridianen 12 und 13 und die gleiche radiale Lage auf. Darüber hinaus ist aus Fig. 3f auch zu erkennen, wie sich die Stufenhöhen der Stufen 19 bis 20 in den beiden senkrecht zueinander stehenden Hauptmeridianen 12 und 13 unterscheiden. Es ist dabei zu erkennen, dass die Stufenhöhen im steilen Hauptmeridian 13 größer sind, als im flachen Hauptmeridian 12. Obwohl somit eine rotationssymmetrische Einhüllende 14, die die Stufenspitzen 15 bis 17 der Stufen 18 bis 20 sowohl in dem steilen als auch in dem flachen Hauptmeridian 13 beziehungsweise 12 verbindet, ist dennoch eine torisch brechende Funktionalität durch die unterschiedlichen Stufenhöhen in den Hauptmeridianen erreicht. Durch diese variierenden Stufenhöhen ist quasi ein torisch brechendes Oberflächenprofil bereitgestellt, auf welchem in azimutaler Richtung umlaufend die Stufenhöhen der Stufen 18 bis 20 variieren. Bei diesem Beispiel sind die Stufen vollständig umlaufend um die Achse A ausgebildet und an keiner azimutalen Stile gleich Null.

Demgegenüber ist in Fig. 3e die Einhüllende 22 gezeigt.

Im Vergleich zwischen Fig. 3e und 3f ist wiederum die Reduktion der Dicke des optischen Teils zu erkennen, wobei in dem Zusammenhang durch d1 die Reduktion eines Ausführungsbeispiels einer erfindungsgemäßen Augenlinse im Vergleich zum Stand der Technik im flachen Hauptmeridian 12 und durch d2 die Reduktion der Dicke des optischen Teils eines Ausführungsbeispiels einer erfindungsgemäßen Augenlinse im Vergleich zum Stand der Technik im steilen Hauptmeridian 13 gezeigt ist. Die Reduktion d1 beträgt vorzugsweise zwischen 0,15 und 0,3, insbesondere im Wesentlichen 0,21. Die Reduktion d2 beträgt vorzugsweise zwischen 0,3 und 0,5, im Wesentlichen vorzugsweise 0,39.

In Fig. 3g ist ein Diagramm gezeigt, in welchem die sagittale Höhendifferenz ΔS in Abhängigkeit vom Radius r des optischen Teils 2 für die aus dem Stand der Technik bekannte, lediglich torische Intraokularlinsen gezeigt ist. Dem gegenüber gestellt ist in dem Diagramm gemäß Fig. 3h die sagittale Höhendifferenz ΔS in Abhängigkeit vom Radius r für ein Ausführungsbeispiel einer erfindungsgemäßen Augenlinse 1 gezeigt. Auch hier ist wieder zu erkennen, dass mit zunehmendem Radius r diese Sagittalhöhendifferenz ΔS abnimmt.

In Fig. 3i ist für das Ausführungsbeispiel gemäß Fig. 3a ff. wiederum die Fokusbreite $B_F$ des tangentialen Fokus FT und des sagittalen Fokus FS gezeigt, wobei hier durch die Distanz der Spitzen der Foki ein Astigmatismus mit etwa 8 Dioptrien charakterisiert ist.

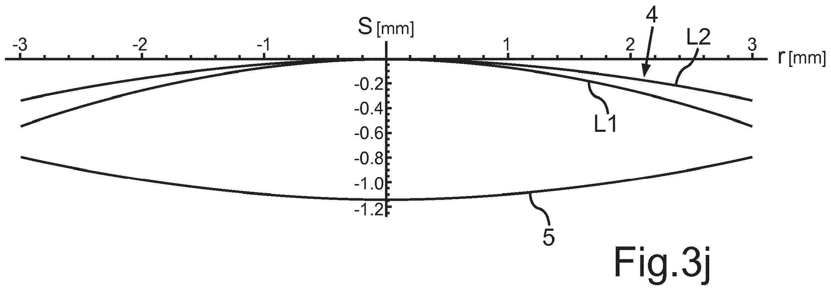

In Fig. 3j ist ein Diagramm gezeigt, in dem die sagittale Höhe in Abhängigkeit vom Radius für das aus dem Stand der Technik bekannte Beispiel einer rein torischen Intraokularlinse gezeigt ist, wie dies auch bereits der Darstellung in Fig. 2l entspricht.

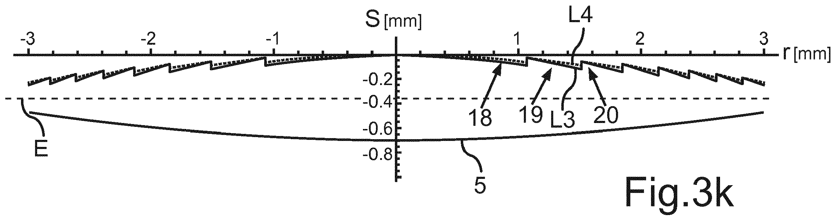

In Fig. 3k ist ein Diagramm gezeigt, in welchem die sagittale Höhe in Abhängigkeit vom Radius für das Ausführungsbeispiel gemäß Fig. 3a ff. gezeigt ist. Auch hier ist die wesentliche Volumenreduktion des optischen Teils 2 zu erkennen. Ebenso ist hier die spiegelsymmetrische Ausgestaltung zur Ebene E, die senkrecht auf der Achse A steht, zu erkennen. Der Konturenverlauf der Seite 5 entspricht dem Konturenverlauf einer Einhüllenden 14, wie sie die Stufenspitzen der Stufen der Kurven L3 und L4, die die gestuften rotationssymmetrischen Profile im steilen Hauptmeridian 13 und im flachen Hauptmeridian 12, charakterisieren.

Bei den bisher erläuterten Ausführungsbeispielen einer erfindungsgemäßen Augenlinse sind die Seiten 4 und 5 konvex gekrümmt.

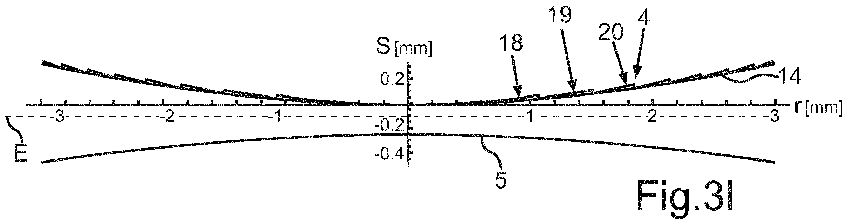

In Fig. 3l ist ein Diagramm gezeigt, in dem die sagittale Höhe in Abhängigkeit vom Radius für ein weiteres Ausführungsbeispiel einer erfindungsgemäßen Augenlinse gezeigt ist. Im Unterschied zu den bisher erläuterten Ausführungen ist hier vorgesehen, dass die Seite 4 und auch die Seite 5 des optischen Teils 2 jeweils konkav gekrümmt sind. Auch hier ist die Ausgestaltung derart, dass die Seite 5 in ihrem Konturenverlauf spiegelsymmetrisch der Einhüllenden 14 bei Spiegelung an der Ebene E ist. Die in Fig. 3l gezeigte Ausgestaltung ist analog einem Ausführungsbeispiel, wie es in Fig. 2a ff. erläutert wurde. Es kann auch darüber hinaus ein beidseitig konkaves Ausführungsbeispiel vorgesehen sein, welches der Ausgestaltung in Fig. 3k entspricht und somit in den senkrecht aufeinander stehenden Hauptmeridianen 12 und 13 radial gestuft ist und unterschiedliche Stufenhöhen in diesen Hauptmeridianen für die Stufen ausgebildet sind.

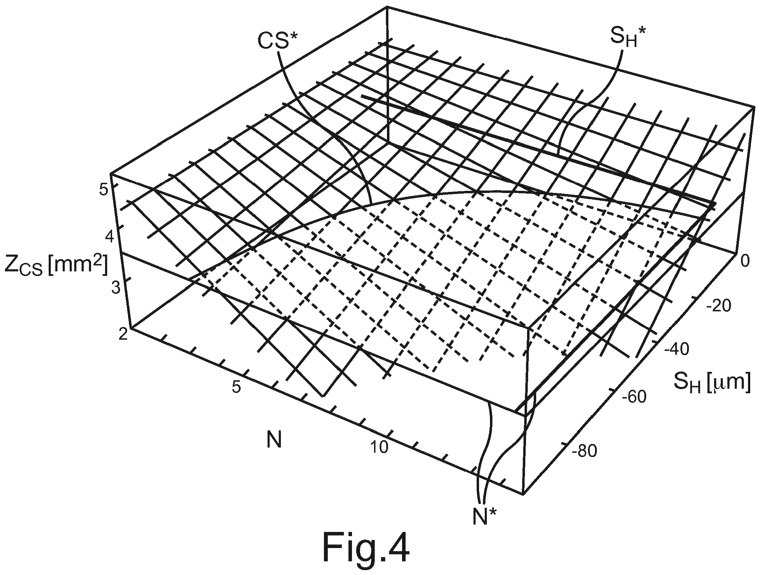

In Fig. 4 ist ein Diagramm gezeigt, in welchem ein Zusammenhang zwischen einer Anzahl von Zonen eines optischen Teils 2, einer Flächengröße eines Querschnitts des optischen Teils, und der Stufenhöhe angegeben ist. Es bezeichnen hierbei $S^*_H$, die bevorzugte Grenze einer minimalen Stufenhöhe, $CS^*$ eine bevorzugte Grenze für den Verlauf der Querschnittfläche und $N^*$ die bevorzugte Grenze für die Zonenzahl. Der Bereich zwischen den Grenzlinien ist bevorzugt für Augenlinsen der Erfindung. Die Querschnittfläche (Schnittebene enthält Achse A) des optischen Teils beträgt vorzugsweise zwischen 3 und 4 mm$^2$, insbesondere zwischen 3, 2 und 3, 8 mm$^2$.

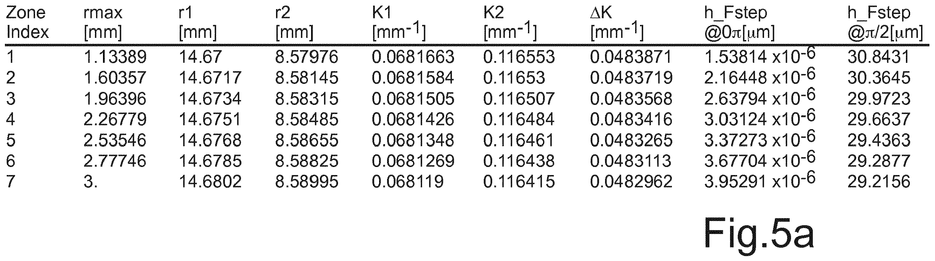

In Fig. 5a ist eine Tabelle für ein weiteres Ausführungsbeispiel einer Augenlinse gezeigt, in welcher für die beispielhafte Anzahl von 7 Zonen von links nach rechts die Parameter des maximale Zonenradius, des Krümmungsradius r1 am flachen Hauptmeridian, des Krümmungsradius r2 am steilen Hauptmeridian, der Krümmung am flachen und am steilen Hauptmeridian, der Krümmungsdifferenz, der Stufenhöhen "h-Fstep $0\pi$" am flachen Hauptmeridian und der Stufenhöhen "h-Fstep $\pi/2$" am steilen Hauptmeridian gezeigt sind. Es ist zu erkennen, dass hier die Stufenhöhen variieren. Im Unterschied zu dem nachfolgenden Beispiel gemäß Fig. 7a ff. ist weiter, dass die zylindrische Brechkraft 6 Dioptrien beträgt und der Krümmungsradius RoC-Face2 10.7383 mm beträgt. Die Verläufe der Kurven der anderen Parameter sind in der Kurvenform gleich, weichen in den Maximalwerten und/oder Minimalwerten ggf. ab.

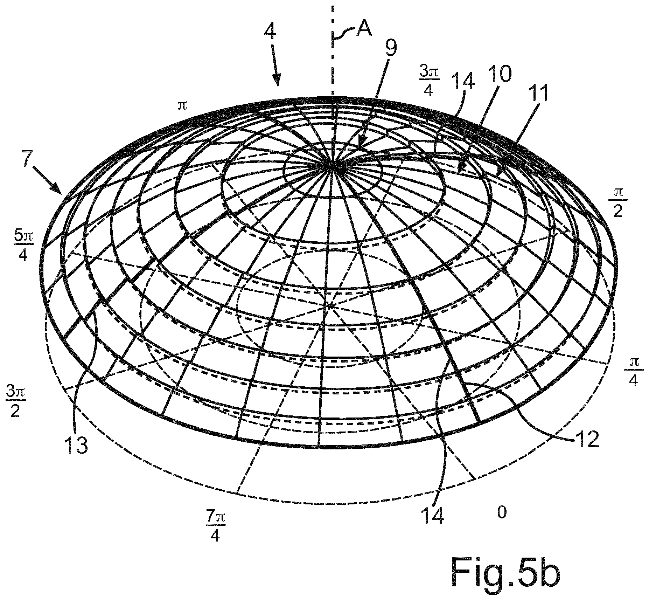

In Fig. 5b ist eine dreidimensionale Oberflächentopographie des Ausführungsbeispiels gezeigt, bei welchem die Oberflächenstruktur 8 und das Oberflächenprofil 7 beispielhaft auf einer Seite 4, und gemeinsam dort ausgebildet sind. Hier ist analog zur Ausführung in Fig. 2m am flachen Hauptmeridian 12 keine Stufung ausgebildet und der Konturenverlauf am flachen Hauptmeridian entspricht vorzugsweise der Einhüllenden am gestuften steilen Hauptmeridian und der Konturenverlauf bzw. die Einhüllenden sind rotationssymmetrisch. Ebenso ist insbesondere eine Spiegelung des rotationssymmetrischen Konturenverlaufs an der Ebene E gleich dem Konturenverlauf der Seite 5.

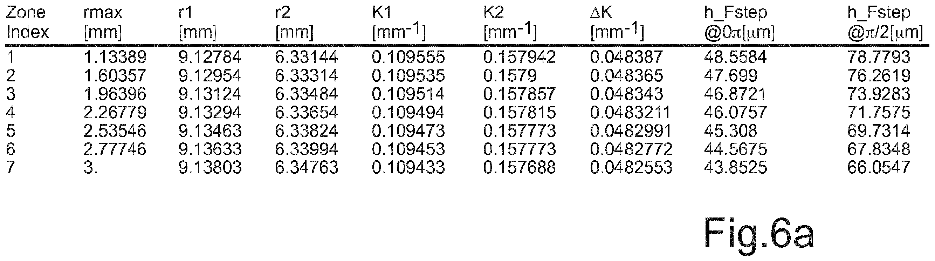

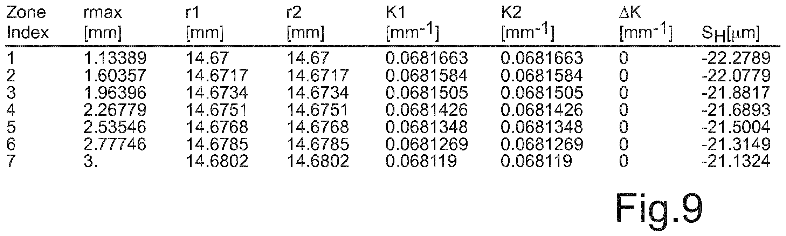

In Fig. 6a ist eine Tabelle vergleichbar mit Fig. 9a für ein weiteres Ausführungsbeispiel einer Augenlinse gezeigt, in welcher für die beispielhafte Anzahl von 7 Zonen von links nach rechts die Parameter des maximale Zonenradius, des Krümmungsradius r1 am flachen Hauptmeridian, des Krümmungsradius r2 am steilen Hauptmeridian, der Krümmung am flachen und am steilen Hauptmeridian, der Krümmungsdifferenz, der Stufenhöhen "h-Fstep $0\pi$" und "h-Fstep $\pi/2$" am flachen und am steilen Hauptmeridian gezeigt sind. Es ist zu erkennen, dass hier die Stufenhöhen variieren. Im Unterschied zu dem nachfolgenden Beispiel gemäß Fig. 9a ff. ist weiter, dass die zylindrische Brechkraft 6 Dioptrien beträgt und der Krümmungsradius RoC-Face2 19.329 mm beträgt. Die Verläufe der Kurven der anderen Parameter sind in der Kurvenform gleich, weichen in den Maximalwerten und/oder Minimalwerten ggf. ab.

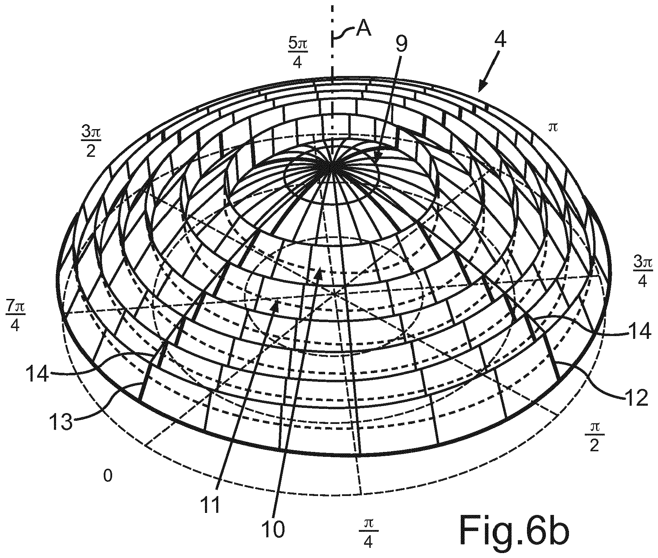

In Fig. 6b ist eine dreidimensionale Oberflächentopographie des Ausführungsbeispiels gezeigt, bei welchem die Oberflächenstruktur 8 und das Oberflächenprofil 7 beispielhaft auf einer Seite 4, und gemeinsam dort ausgebildet sind. Hier ist analog zur Ausführung in Fig. 3k am flachen Hauptmeridian 12 und am steilen Hauptmeridian 13 eine Stufung ausgebildet und der Konturenverlauf der Einhüllenden am flachen Hauptmeridian entspricht vorzugsweise der Einhüllenden am gestuften steilen Hauptmeridian und der Konturenverlauf bzw. die Einhüllenden sind vorzugsweise rotationssymmetrisch. Ebenso ist insbesondere eine Spiegelung des rotationssymmetrischen Konturenverlaufs an der Ebene E gleich dem Konturenverlauf der Seite 5.

In Fig. 7a bis 7m ist ein weiteres Ausführungsbeispiel einer Augenlinse 1 gezeigt, bei welchem eine gestufte Oberflächenstruktur 8 ausgebildet ist, die vollständig umlaufend ausgebildet ist. Diese ist zusätzlich zu einem torisch brechenden Oberflächenprofil ausgebildet. Es ist hier eine Ausgestaltung realisiert, bei der eine Einhüllende keine Rotationssymmetrie aufweist.

Beispielhafte Parameterwerte für die sphärische Brechkraft $F_{Sphäre}$, die zylindrische Brechkraft $F_{Cylinder}$, die Mittendicke CTVA, den Krümmungsradius RoC-Face2 (also $r_S$), insbesondere dem Scheitelkrümmungsradius auf der unstrukturierten Seite und somit auf der Seite des optischen Teils 2, auf welchem die Oberflächenstruktur 8 und das Oberflächenprofil 7 nicht ausgebildet sind, dem maximalen Radius $r_{max}$ des optischen Teils 2, dem Brechungsindex des Immersionsmediums (Kammerwasser) und dem Brechungsindex des Materials des optischen Teils 2, der Anzahl "Number of FZones" der Zonen mit Stufen, und der Stufenhöhe $S_H$ ("Step Height")

In Fig. 7a ist der azimutale Verlauf der Gesamtbrechkraft gezeigt. In Fig. 7b und 7c ist der azimutale Verlauf der sphärischen Brechkraft und der zylindrischen Brechkraft gezeigt.

In Fig. 7d und Fig. 7e ist der azimutale Verlauf der Brechkraft $F_{Face1}$ auf der Seite des optischen Teils, auf welcher die Oberflächenstruktur 8 und das Oberflächenprofil 7 ausgebildet sind (Fig. 7d), und der azimutale Verlauf der Brechkraft $F_{Face2}$ der gegenüberliegenden Seite (Fig. 7e) gezeigt.

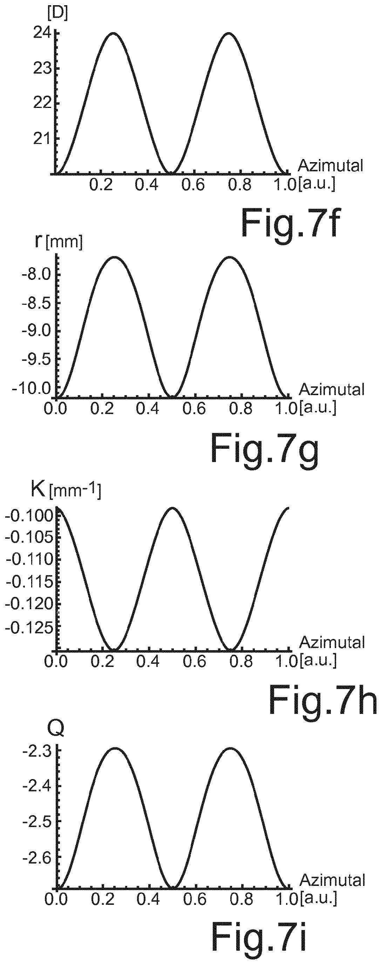

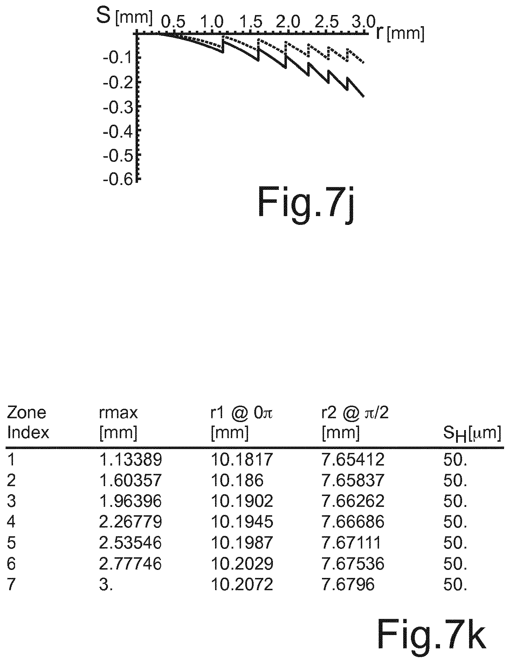

In Fig. 7f ist der Verlauf der Gesamtbrechkraft in Abhängigkeit des normierten Azimutwinkels gezeigt. In Fig. 7g ist der Verlauf des Scheitelradius in Abhängigkeit des normierten Azimutwinkels gezeigt. In Fig. 7h ist der Verlauf der Krümmung K in Abhängigkeit des normierten Azimutwinkels gezeigt. In Fig. 7i ist der Verlauf der konischen Konstante k bzw. Q in Abhängigkeit des normierten Azimutwinkels gezeigt. In Fig. 7j ist der Verlauf der der sagittalen Höhe S am flachen und steilen Hauptmeridian in Abhängigkeit des normierten Azimutwinkels gezeigt.

In Fig. 7k ist eine Tabelle gezeigt, in welcher für die beispielhafte Anzahl von 7 Zonen von links nach rechts die Parameter des maximale Zonenradius, des Krümmungsradius r1 am flachen Hauptmeridian, des Krümmungsradius r2 am steilen Hauptmeridian, und der Stufenhöhe der Stufen angegeben ist. Es ist zu erkennen, dass hier die Stufenhöhe konstant ist.

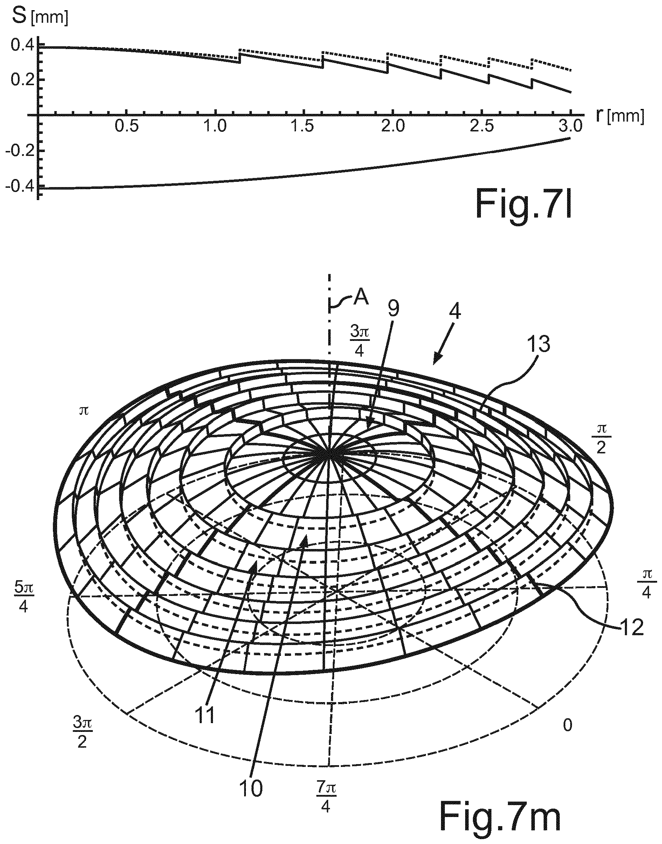

In Fig. 7l ist ein Diagramm gezeigt, in welchem die sagittale Höhe in Abhängigkeit vom Radius r des optischen Teils 2 angegeben ist, wobei hier sowohl der Verlauf der Rückseite 5 als auch der Verläufe des flachen und des steilen Hauptmeridians, die hier jeweils gestuft sind, auf der Vorderseite 4, gezeigt sind.

In Fig. 7m ist eine dreidimensionale Oberflächentopographie des Ausführungsbeispiels gezeigt, bei welchem die Oberflächenstruktur 8 und das Oberflächenprofil 7 beispielhaft auf einer Seite 4, und gemeinsam dort ausgebildet sind.

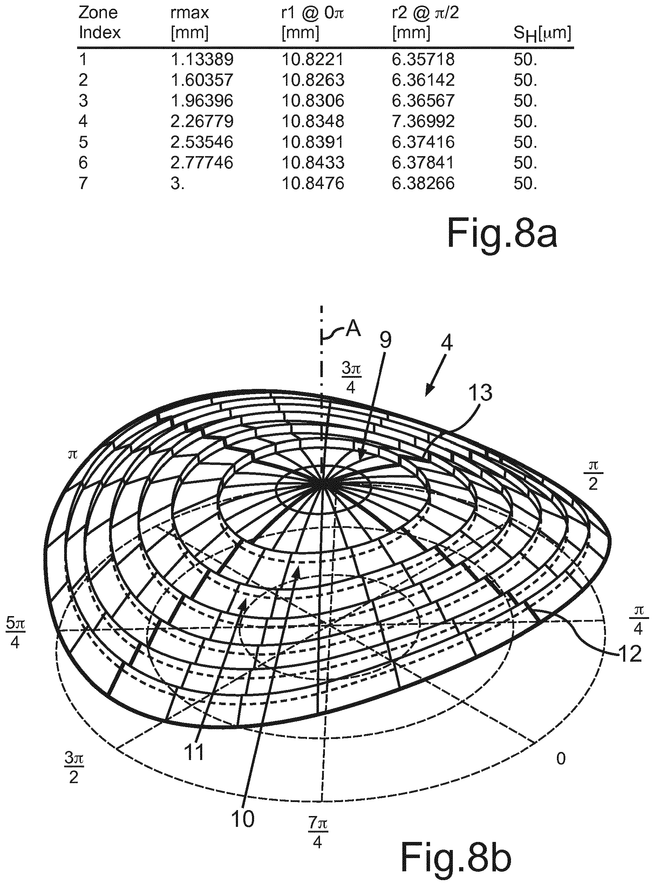

In Fig. 8a ist eine Tabelle vergleichbar mit Fig. 7k für ein weiteres Ausführungsbeispiel einer Augenlinse gezeigt, in welcher für die beispielhafte Anzahl von 7 Zonen von links nach rechts die Parameter des maximale Zonenradius, des Krümmungsradius r1 am flachen Hauptmeridian, des Krümmungsradius r2 am steilen Hauptmeridian, und der Stufenhöhe der Stufen angegeben ist. Es ist zu erkennen, dass hier die Stufenhöhe konstant ist. Im Unterschied zu dem Beispiel gemäß Fig. 7a ff. ist weiter, dass die zylindrische Brechkraft 8 Dioptrien beträgt und der Krümmungsradius "RoC-Face2" 14.4027 mm beträgt. Die Verläufe der Kurven der anderen Parameter sind in der Kurvenform gleich, weichen in den Maximalwerten und/oder Minimalwerten ggf. ab.

In Fig. 8b ist eine dreidimensionale Oberflächentopographie des Ausführungsbeispiels gezeigt, bei welchem die Oberflächenstruktur 8 und das Oberflächenprofil 7 beispielhaft auf einer Seite 4, und gemeinsam dort ausgebildet sind.

In Fig. 9 ist eine Tabelle vergleichbar mit Fig. 5a für ein weiteres Ausführungsbeispiel einer Augenlinse gezeigt, in welcher für die beispielhafte Anzahl von 7 Zonen von links nach rechts die Parameter des maximale Zonenradius, des Krümmungsradius r1 am flachen Hauptmeridian, des Krümmungsradius r2 am steilen Hauptmeridian, und der Stufenhöhe der Stufen angegeben ist. Es ist zu erkennen, dass hier die Stufenhöhe $S_H$ variiert. Im Unterschied zu dem Beispiel gemäß Fig. 5a ff. ist weiter, dass die zylindrische Brechkraft 6 Dioptrien beträgt. Die Verläufe der Kurven der anderen Parameter sind in der Kurvenform gleich, weichen in den Maximalwerten und/oder Minimalwerten ggf. ab.

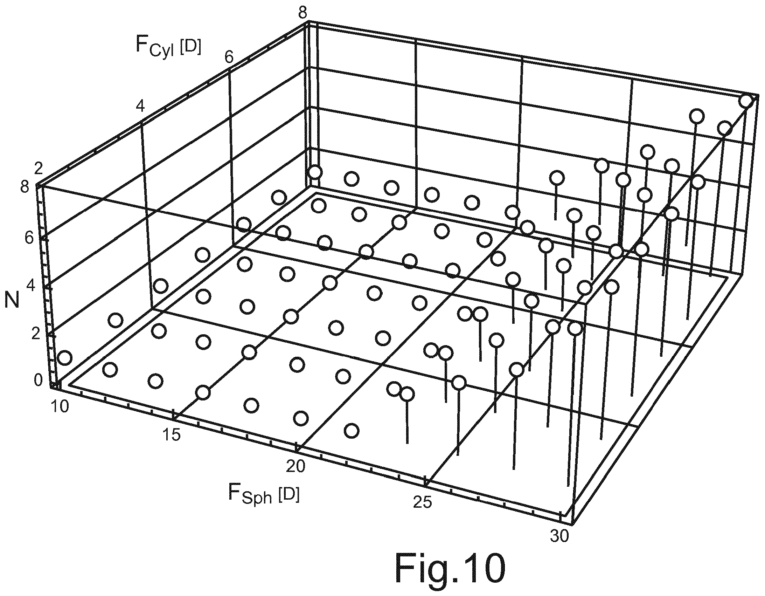

In Fig. 10 ist ein Diagramm gezeigt, in welchem ein Zusammenhang zwischen einer Anzahl N der eine radial gestufte Oberflächenstruktur erzeugenden ringförmigen optischen Zonen, einer zylindrischen Brechkraft $F_{cyl}$, die durch das torisch brechende Oberflächenprofil erzeugt und zur Gesamtbrechkraft beigetragen wird, und einer sphärischen Brechkraft $F_{sph}$ für Ausführungsbeispiele von erfindungsgemäßen Augenlinsen gezeigt ist. Die Werte sind auf Basis eines Brechungsindexwerts zwischen 1,45 und 1,55, insbesondere 1,5, für das Material des optischen Teils der Augenlinse bestimmt.

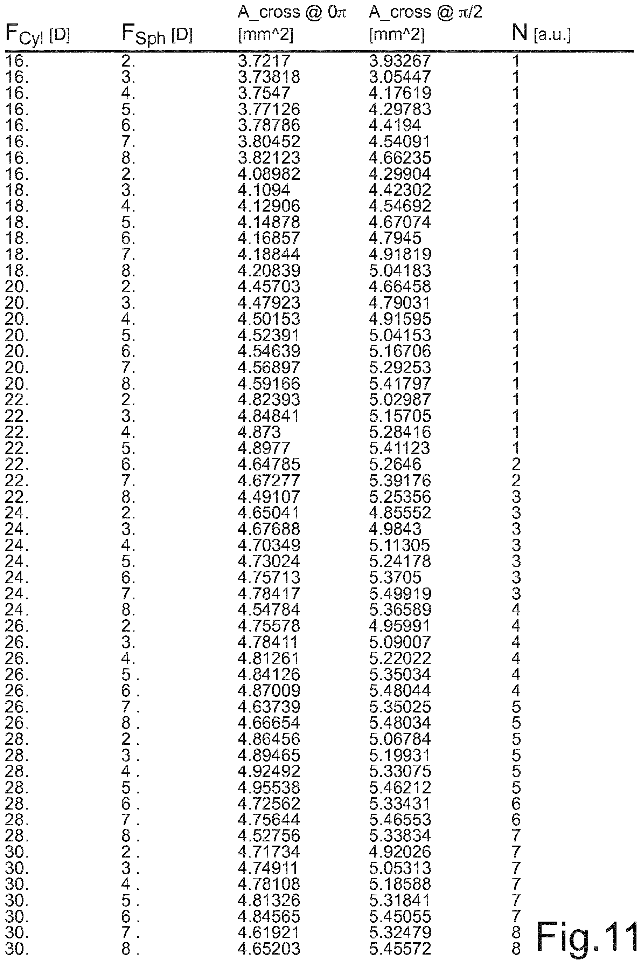

In Fig. 11 ist eine Tabelle gezeigt, in welcher für unterschiedliche sphärische Brechkräfte $F_{sph}$, unterschiedliche zylindrische Brechkräfte $F_{cyl}$, unterschiedliche Zonenzahl N jeweilige Querschnittflächen "A_cross" in $mm^2$ des optischen Teils für Ausführungsbeispiele erfindungsgemäßer Augenlinsen in zwei senkrecht aufeinander stehenden Querschnittebenen angegeben sind. Es bezieht sich dabei auf die Hauptmeridiane bei Null und $\pi/2$. Die Zonenzahl N ist hier bis 8 angegeben, sie kann aber auch höher sein.

## Patentansprüche

1. Augenlinse (1) mit einem optischen Teil (2), der eine in Richtung einer optischen Hauptachse (A) der Augenlinse (1) betrachtet erste optische Seite (4) und eine gegenüberliegende zweite optische Seite (5) aufweist, und auf zumindest einer der beiden Seiten (4, 5) ein torisch brechendes Oberflächenprofil (7) ausgebildet ist, wobei die Augenlinse (1) zusätzlich zum torisch brechenden Oberflächenprofil (7) eine in radialer Richtung des optischen Teils (2) gestufte Oberflächenstruktur (8) aufweist, und die gestufte Oberflächenstruktur (8) auf zumindest einer Seite (4, 5) ausgebildet ist, **dadurch gekennzeichnet, dass** ein radialer Konturenverlauf einer Einhüllenden (14), die auf einer Seite (4, 5) ausgebildete Stufenspitzen (15 bis 17) von die gestufte Oberflächenstruktur (8) bildenden Stufen (18 bis 20) verbindet, zumindest abschnittweise einem an einer auf der optischen Hauptachse (A) senkrecht stehenden Symmetrieebene (E) der Augenlinse (1) gespiegelten Konturenverlauf der Oberfläche der gegenüberliegenden anderen Seite (4, 5) entspricht.

2.  Augenlinse (1) nach Anspruch 1, **dadurch gekennzeichnet, dass** das torisch brechende Oberflächenprofil (7) und die gestufte Oberflächenstruktur (8) auf einer gemeinsamen Seite (4, 5) ausgebildet und überlagert sind.

3.  Augenlinse (1) nach Anspruch 1, **dadurch gekennzeichnet, dass** das torisch brechende Oberflächenprofil (7) auf einer Seite (4, 5) und die gestufte Oberflächenstruktur (8) auf der anderen Seite (4, 5) ausgebildet sind.

4.  Augenlinse (1) nach einem der vorhergehenden Ansprüche, **dadurch gekennzeichnet, dass** ein radialer Konturenverlauf eines flachen Hauptmeridians (12) des torisch brechenden Oberflächenprofils (7) gleich einem Konturenverlauf einer Einhüllenden (14) ist, die Stufenspitzen (15 bis 17) von die gestufte Oberflächenstruktur (8) in einem steilen Hauptmeridian (13) des torisch brechenden Oberflächenprofils (7) bildenden Stufen (18 bis 20) verbindet.

5.  Augenlinse (1) nach Anspruch 4, **dadurch gekennzeichnet, dass** ein radialer Konturenverlauf einer Einhüllenden (14), die Stufenspitzen (15 bis 17) von die gestufte Oberflächenstruktur (8) in einem flachen Hauptmeridian (12) des torisch brechenden Oberflächenprofils (8) bildenden Stufen (18 bis 20) verbindet, gleich einem Konturenverlauf einer Einhüllenden (14) ist, die Stufenspitzen (15 bis 17) von die gestufte Oberflächenstruktur (8) in einem steilen Hauptmeridian (13) des torisch brechenden Oberflächenprofils (7) bildenden Stufen (18 bis 20) verbindet.

6.  Augenlinse (1) nach einem der vorhergehenden Ansprüche, **dadurch gekennzeichnet, dass** die Konturenverläufe rotationssymmetrisch um die Hauptachse (A) sind.

7.  Augenlinse (1) nach einem der vorhergehenden Ansprüche, **dadurch gekennzeichnet, dass** Stufenspitzen (15 bis 17) von die gestufte Oberflächenstruktur (8) in einem flachen Hauptmeridian (12) des torisch brechenden Oberflächenprofils (8) bildenden Stufen (18 bis 20) radial an gleicher Stelle ausgebildet sind, wie Stufenspitzen (15 bis 17) von die gestufte Oberflächenstruktur (8) in einem steilen Hauptmeridian (13) des torisch brechenden Oberflächenprofils (7) bildenden Stufen (18 bis 20).

8.  Augenlinse (1) nach einem der vorhergehenden Ansprüche, **dadurch gekennzeichnet, dass** Stufenhöhen von die gestufte Oberflächenstruktur (8) in einem flachen Hauptmeridian (12) des torisch brechenden Oberflächenprofils (8) bildenden Stufen (18 bis 20) unterschiedlich sind, als Stufenhöhen von die gestufte Oberflächenstruktur (8) in einem steilen Hauptmeridian (13) des torisch brechenden Oberflächenprofils (7) bildenden Stufen (18 bis 20).

9.  Augenlinse (1) nach Anspruch 8, **dadurch gekennzeichnet, dass** Stufenhöhen von die gestufte Oberflächenstruktur (8) in einem flachen Hauptmeridian (12) des torisch brechenden Oberflächenprofils (8) bildenden Stufen (18 bis 20) niedriger sind, als Stufenhöhen von die gestufte Oberflächenstruktur (8) in einem steilen Hauptmeridian (13) des torisch brechenden Oberflächenprofils (7) bildenden Stufen (18 bis 20).

10. Augenlinse (1) nach einem der vorhergehenden Ansprüche, **dadurch gekennzeichnet, dass** Stufenhöhen von die gestufte Oberflächenstruktur (8) bildenden Stufen (18 bis 20) nur in einem Hauptmeridian des torisch brechenden Oberflächenprofils (7) gleich Null sind.

11. Augenlinse (1) nach einem der vorhergehenden Ansprüche, **dadurch gekennzeichnet, dass** die erste Seite (4) und die zweite Seite (5) jeweils konvex ausgebildet sind.

12. Augenlinse (1) nach einem der vorhergehenden Ansprüche 1 bis 7, **dadurch gekennzeichnet, dass** die erste Seite (4) und die zweite Seite (5) jeweils konkav ausgebildet sind.

13. Augenlinse (1) nach einem der vorhergehenden Ansprüche, **dadurch gekennzeichnet, dass** ein sphärischer Brechkraftanteil der Gesamtbrechkraft der Augenlinse (1) jeweils hälftig auf die erste Seite (4) und die zweite Seite (5) verteilt ist oder ein sphärischer Brechkraftanteil der Gesamtbrechkraft der Augenlinse zu 25% bis 35% auf eine Seite (4, 5) und restlich auf die andere Seite (4, 5) verteilt ist.

14. Augenlinse (1) nach einem der vorhergehenden Ansprüche, **dadurch gekennzeichnet, dass** ein Differenzwert zwischen einer Stufenhöhe einer Stufe (18 bis 20) an einem steilen Hauptmeridian (13) und einer Stufenhöhe der Stufe (18 bis 20) an einem flachen Hauptmeridian (12) des torisch brechenden Oberflächenprofils (7) abnimmt, je weiter radial außen liegend die betrachtete Stufe (18 bis 20) in der Oberflächenstruktur (8) ausgebildet ist.

15. Augenlinse (1) nach einem der vorhergehenden Ansprüche, **dadurch gekennzeichnet, dass** die Anzahl der die gestufte Oberflächenstruktur (8) bildenden, zumindest teilweise um die Hauptachse (A) umlaufenden ringförmigen Zonen (18 bis 20) zwischen 5 und 14 beträgt.

**Claims**

1. Lens (1) for an eye, comprising an optical part (2), which has a first optical side (4) when viewed in the direction of an optical principal axis (A) of the lens (1) for an eye and an opposing second optical side (5), and having formed on at least one of the two sides (4, 5) a surface profile (7) that is refractive in toric fashion, wherein, in addition to the surface profile (7) that is refractive in toric fashion, the lens (1) for an eye has a surface structure (8) that is stepped in the radial direction of the optical part (2), the stepped surface structure (8) being formed on at least one side (4, 5), **characterized in that** a radial contour profile of an envelope (14) which connects step tips, (15 to 17) of steps (18 to 20) forming the stepped surface structure (8), formed on one side (4, 5) corresponds at least in sections to a contour profile, of the surface of the opposite other side (4, 5), which has been mirrored in a plane of symmetry (E), perpendicular to the optical principal axis (A), of the lens (1) for an eye.

2. Lens (1) for an eye according to Claim 1, **characterized in that** the surface profile (7) that is refractive in toric fashion and the stepped surface structure (8) are formed and overlaid on a common side (4, 5).

3. Lens (1) for an eye according to Claim 1, **characterized in that** the surface profile (7) that is refractive in toric fashion is formed on one side (4, 5) and the stepped surface structure (8) is formed on the other side (4, 5).

4. Lens (1) for an eye according to any one of the preceding claims, **characterized in that** a radial contour profile of a flat principal meridian (12) of the surface profile (7) that is refractive in toric fashion is the same as a contour profile of an envelope (14) which connects the step tips (15 to 17) of steps (18 to 20) forming the stepped surface structure (8) in a steep principal meridian (13) of the surface profile (7) that is refractive in toric fashion.

5. Lens (1) for an eye according to Claim 4, **characterized in that** a radial contour profile of an envelope (14) which connects the step tips (15 to 17) of steps (18 to 20) forming the stepped surface structure (8) in a flat principal meridian (12) of the surface profile (8) that is refractive in toric fashion is the same as a contour profile of an envelope (14) which connects the step tips (15 to 17) of steps (18 to 20) forming the stepped surface structure (8) in a steep principal meridian (13) of the surface profile (7) that is refractive in toric fashion.

6. Lens (1) for an eye according to any one of the preceding claims, **characterized in that** the contour profiles are rotationally symmetric about the principal axis (A).

7. Lens (1) for an eye according to any one of the preceding claims, **characterized in that** step tips (15 to 17) of steps (18 to 20) forming the stepped surface structure (8) in a flat principal meridian (12) of the surface profile (8) that is refractive in toric fashion are formed radially at the same point as step tips (15 to 17) of steps (18 to 20) forming the stepped surface structure (8) in a steep principal meridian (13) of the surface profile (7) that is refractive in toric fashion.

8. Lens (1) for an eye according to any one of the preceding claims, **characterized in that** step heights of steps (18 to 20) forming the stepped surface structure (8) in a flat principal meridian (12) of the surface profile (8) that is refractive in toric fashion are different from step heights of steps (18 to 20) forming the stepped surface structure (8) in a steep principal meridian (13) of the surface profile (7) that is refractive in toric fashion.

9. Lens (1) for an eye according to Claim 8, **characterized in that** step heights of steps (18 to 20) forming the stepped surface structure (8) in a flat principal meridian (12) of the surface profile (8) that is refractive in toric fashion are lower than step heights of steps (18 to 20) forming the stepped surface structure (8) in a steep principal meridian (13) of the surface profile (7) that is refractive in toric fashion.

**10.** Lens (1) for an eye according to any one of the preceding claims, **characterized in that** step heights of steps (18 to 20) forming the stepped surface structure (8) are equal to zero only in one principal meridian of the surface profile (7) that is refractive in toric fashion.

**11.** Lens (1) for an eye according to any one of the preceding claims, **characterized in that** the first side (4) and the second side (5) each have a convex form.

**12.** Lens (1) for an eye according to any one of the preceding Claims 1 to 7, **characterized in that** the first side (4) and the second side (5) each have a concave form.

**13.** Lens (1) for an eye according to any one of the preceding claims, **characterized in that** a spherical refractive power component of the overall refractive power of the lens (1) for an eye is distributed so that the first side (4) and the second side (5) each have half or a spherical refractive power component of the overall refractive power of the lens for an eye is distributed with 25% to 35% on one side (4, 5) and the rest on the other side (4, 5).

**14.** Lens (1) for an eye according to any one of the preceding claims, **characterized in that** a difference value between a step height of a step (18 to 20) at a steep principal meridian (13) and a step height of the step (18 to 20) at a flat principal meridian (12) of the surface profile (7) that is refractive in toric fashion reduces, the further the considered step (18 to 20) is formed radially to the outside in the surface structure (8) .

**15.** Lens (1) for an eye according to any one of the preceding claims, **characterized in that** the number of ring-shaped zones (18 to 20) that form the stepped surface structure (8) and at least partly extend around the principal axis (A) is between 5 and 14.

## Revendications

**1.** Lentille ophtalmique (1) munie d'une partie optique (2) qui présente, vus dans la direction d'un axe principal optique (A) de la lentille ophtalmique (1), un premier côté optique (4) et un deuxième côté optique opposé (5), et un profil de surface à réfraction torique (7) est formé sur au moins un des deux côtés (4, 5), la lentille ophtalmique (1) présentant, outre le profil de surface à réfraction torique (7), une structure de surface échelonnée (8) dans la direction radiale de la partie optique (2), et la structure de surface échelonnée (8) étant formée sur au moins un côté (4, 5), **caractérisée en ce qu'**une allure de contour radiale d'une enveloppe (14), qui relie des pointes d'échelon (15 à 17) formées sur un côté (4, 5) d'échelons (18 à 20) formant la structure de surface échelonnée (8), correspond au moins en section à une allure de contour, réfléchie sur un plan de symétrie (E) perpendiculaire à l'axe principal optique (A) de la lentille ophtalmique (1), de la surface de l'autre côté opposé (4, 5).

**2.** Lentille ophtalmique (1) selon la revendication 1, **caractérisée en ce que** le profil de surface à réfraction torique (7) et la structure de surface échelonnée (8) sont formés sur un côté commun (4, 5) et superposés.

**3.** Lentille ophtalmique (1) selon la revendication 1, **caractérisée en ce que** le profil de surface à réfraction torique (7) est formé sur un côté (4, 5) et la structure de surface échelonnée (8) est formée sur l'autre côté (4, 5).

**4.** Lentille ophtalmique (1) selon l'une quelconque des revendications précédentes, **caractérisée en ce qu'**une allure de contour radiale d'un méridien principal plat (12) du profil de surface à réfraction torique (7) est identique à une allure de contour d'une enveloppe (14), qui relie des pointes d'échelon (15 à 17) d'échelons (18 à 20) formant la structure de surface échelonnée (8) dans un méridien principal abrupt (13) du profil de surface à réfraction torique (7) .

**5.** Lentille ophtalmique (1) selon la revendication 4, **caractérisée en ce qu'**une allure de contour radiale d'une enveloppe (14), qui relie des pointes d'échelon (15 à 17) d'échelons (18 à 20) formant la structure de surface échelonnée (8) dans un méridien principal plat (12) du profil de surface à réfraction torique (8), est identique à une allure de contour d'une enveloppe (14), qui relie des pointes d'échelon (15 à 17) d'échelons (18 à 20) formant la structure de surface échelonnée (8) dans un méridien principal abrupt (13) du profil de surface à réfraction torique (7).

**6.** Lentille ophtalmique (1) selon l'une quelconque des revendications précédentes, **caractérisée en ce que** les allures de contour sont symétriques par rotation autour de l'axe principal (A).

**7.** Lentille ophtalmique (1) selon l'une quelconque des revendications précédentes, **caractérisée en ce que** des pointes d'échelon (15 à 17) d'échelons (18 à 20) formant la structure de surface échelonnée (8) dans un méridien principal plat (12) du profil de surface à réfraction torique (8) sont formées radialement au même emplacement que des pointes d'échelon (15 à 17) d'échelons (18 à 20) formant la structure de surface échelonnée (8) dans un méridien principal abrupt (13) du profil de surface à réfraction torique (7).

**8.** Lentille ophtalmique (1) selon l'une quelconque des revendications précédentes, **caractérisée en ce que** des hauteurs d'échelon d'échelons (18 à 20) formant la structure de surface échelonnée (8) dans un méridien principal plat (12) du profil de surface à réfraction torique (8) sont différentes de hauteurs d'échelon d'échelons (18 à 20) formant la structure de surface échelonnée (8) dans un méridien principal abrupt (13) du profil de surface à réfraction torique (7).

**9.** Lentille ophtalmique (1) selon la revendication 8, **caractérisée en ce que** des hauteurs d'échelon d'échelons (18 à 20) formant la structure de surface échelonnée (8) dans un méridien principal plat (12) du profil de surface à réfraction torique (8) sont inférieures à des hauteurs d'échelon d'échelons (18 à 20) formant la structure de surface échelonnée (8) dans un méridien principal abrupt (13) du profil de surface à réfraction torique (7).

**10.** Lentille ophtalmique (1) selon l'une quelconque des revendications précédentes, **caractérisée en ce que** des hauteurs d'échelon d'échelons (18 à 20) formant la structure de surface échelonnée (8) sont égales à zéro uniquement dans un méridien principal du profil de surface à réfraction torique (7).

**11.** Lentille ophtalmique (1) selon l'une quelconque des revendications précédentes, **caractérisée en ce que** le premier côté (4) et le deuxième côté (5) sont chacun configurés sous forme convexe.

**12.** Lentille ophtalmique (1) selon l'une quelconque des revendications 1 à 7 précédentes, **caractérisée en ce que** le premier côté (4) et le deuxième côté (5) sont chacun configurés sous forme concave.

**13.** Lentille ophtalmique (1) selon l'une quelconque des revendications précédentes, **caractérisée en ce qu'**une fraction sphérique de pouvoir de réfraction du pouvoir de réfraction total de la lentille ophtalmique (1) est répartie respectivement par moitié sur le premier côté (4) et le deuxième côté (5), ou une fraction sphérique de pouvoir de réfraction du pouvoir de réfraction total de la lentille ophtalmique est répartie à hauteur de 25 % à 35 % sur un côté (4, 5) et le reste sur l'autre côté (4, 5).

**14.** Lentille ophtalmique (1) selon l'une quelconque des revendications précédentes, **caractérisée en ce qu'**une valeur de différence entre une hauteur d'échelon d'un échelon (18 à 20) sur un méridien principal abrupt (13) et une hauteur d'échelon de l'échelon (18 à 20) sur un méridien principal plat (12) du profil de surface à réfraction torique (7) diminue plus l'échelon en question (18 à 20) est formé radialement à l'extérieur dans la structure de surface (8).

**15.** Lentille ophtalmique (1) selon l'une quelconque des revendications précédentes, **caractérisée en ce que** le nombre de zones de forme annulaire (18 à 20) formant la structure de surface échelonnée (8), au moins partiellement circonférentielles autour de l'axe principal (A), est compris entre 5 et 14.

Fig.1a

Fig.1b

Fig.2a

Fig.2b

Fig.2c

Fig.2d

Fig.2e

Fig.2f

Fig.2g

Fig.2h

Fig.2i

Fig.2j

Fig.2k

Fig.2l

Fig.2m

EP 3 033 042 B1

F_Ges

Fig.3a

F_Cyl(φ)

Fig.3b

F_Sphäre

Fig.3c

r_S [mm]

Fig.3d

Fig.3e

Fig.3f

Fig.3g

Fig.3h

Fig.3i

Fig.3j

EP 3 033 042 B1

Fig.3k

EP 3 033 042 B1

Fig.3I

Fig.4

EP 3 033 042 B1

| Zone Index | rmax [mm] | r1 [mm] | r2 [mm] | K1 [mm$^{-1}$] | K2 [mm$^{-1}$] | $\Delta$K [mm$^{-1}$] | h_Fstep @0$\pi$[$\mu$m] | h_Fstep @$\pi$/2[$\mu$m] |
|---|---|---|---|---|---|---|---|---|
| 1 | 1.13389 | 14.67 | 8.57976 | 0.0681663 | 0.116553 | 0.0483871 | 1.53814 x10$^{-6}$ | 30.8431 |
| 2 | 1.60357 | 14.6717 | 8.58145 | 0.0681584 | 0.11653 | 0.0483719 | 2.16448 x10$^{-6}$ | 30.3645 |
| 3 | 1.96396 | 14.6734 | 8.58315 | 0.0681505 | 0.116507 | 0.0483568 | 2.63794 x10$^{-6}$ | 29.9723 |
| 4 | 2.26779 | 14.6751 | 8.58485 | 0.0681426 | 0.116484 | 0.0483416 | 3.03124 x10$^{-6}$ | 29.6637 |
| 5 | 2.53546 | 14.6768 | 8.58655 | 0.0681348 | 0.116461 | 0.0483265 | 3.37273 x10$^{-6}$ | 29.4363 |
| 6 | 2.77746 | 14.6785 | 8.58825 | 0.0681269 | 0.116438 | 0.0483113 | 3.67704 x10$^{-6}$ | 29.2877 |
| 7 | 3. | 14.6802 | 8.58995 | 0.068119 | 0.116415 | 0.0482962 | 3.95291 x10$^{-6}$ | 29.2156 |

## Fig.5a

Fig.5b

| Zone Index | rmax [mm] | r1 [mm] | r2 [mm] | K1 [mm$^{-1}$] | K2 [mm$^{-1}$] | $\Delta$K [mm$^{-1}$] | h_Fstep @0$\pi$[$\mu$m] | h_Fstep @$\pi$/2[$\mu$m] |
|---|---|---|---|---|---|---|---|---|
| 1 | 1.13389 | 9.12784 | 6.33144 | 0.109555 | 0.157942 | 0.048387 | 48.5584 | 78.7793 |
| 2 | 1.60357 | 9.12954 | 6.33314 | 0.109535 | 0.1579 | 0.048365 | 47.699 | 76.2619 |
| 3 | 1.96396 | 9.13124 | 6.33484 | 0.109514 | 0.157857 | 0.048343 | 46.8721 | 73.9283 |
| 4 | 2.26779 | 9.13294 | 6.33654 | 0.109494 | 0.157815 | 0.0483211 | 46.0757 | 71.7575 |
| 5 | 2.53546 | 9.13463 | 6.33824 | 0.109473 | 0.157773 | 0.0482991 | 45.308 | 69.7314 |
| 6 | 2.77746 | 9.13633 | 6.33994 | 0.109453 | 0.157773 | 0.0482772 | 44.5675 | 67.8348 |
| 7 | 3. | 9.13803 | 6.34763 | 0.109433 | 0.157688 | 0.0482553 | 43.8525 | 66.0547 |

# Fig.6a

Fig.6b

F_Sphäre = 20. D
F_Cylinder = 4. D
CTVA = 1 mm
RoC_Face 2 = 15.7219 mm
rmax = 3 mm
nmed = 1.336
niol = 1.46
Number of FZones = 7
Step Height = 50. µm

$F_{Ges}$

[D]

Azimut [rad]

## Fig.7a

$F_{Sphäre}$

[D]

Azimut [rad]

## Fig.7b

$F_{Cyl(\phi)}$

[D]

Azimut [rad]

## Fig.7c

$F_{Face\ 1}$

[D]

Azimutal [a.u.]

## Fig.7d

$F_{Face\ 2}$

[D]

Azimutal [a.u.]

## Fig.7e

Fig.7f

Fig.7g

Fig.7h

Fig.7i

Fig.7j

| Zone Index | rmax [mm] | r1 @ 0π [mm] | r2 @ π/2 [mm] | $S_H$[µm] |
|---|---|---|---|---|
| 1 | 1.13389 | 10.1817 | 7.65412 | 50. |
| 2 | 1.60357 | 10.186 | 7.65837 | 50. |
| 3 | 1.96396 | 10.1902 | 7.66262 | 50. |
| 4 | 2.26779 | 10.1945 | 7.66686 | 50. |
| 5 | 2.53546 | 10.1987 | 7.67111 | 50. |
| 6 | 2.77746 | 10.2029 | 7.67536 | 50. |
| 7 | 3. | 10.2072 | 7.6796 | 50. |

Fig.7k

Fig.7l

Fig.7m

| Zone Index | rmax [mm] | r1 @ 0π [mm] | r2 @ π/2 [mm] | $S_H$[μm] |
|---|---|---|---|---|
| 1 | 1.13389 | 10.8221 | 6.35718 | 50. |
| 2 | 1.60357 | 10.8263 | 6.36142 | 50. |
| 3 | 1.96396 | 10.8306 | 6.36567 | 50. |
| 4 | 2.26779 | 10.8348 | 7.36992 | 50. |
| 5 | 2.53546 | 10.8391 | 6.37416 | 50. |
| 6 | 2.77746 | 10.8433 | 6.37841 | 50. |
| 7 | 3. | 10.8476 | 6.38266 | 50. |

## Fig.8a

## Fig.8b

| Zone Index | rmax [mm] | r1 [mm] | r2 [mm] | K1 [mm$^{-1}$] | K2 [mm$^{-1}$] | $\Delta$K [mm$^{-1}$] | $S_H$[μm] |
|---|---|---|---|---|---|---|---|
| 1 | 1.13389 | 14.67 | 14.67 | 0.0681663 | 0.0681663 | 0 | -22.2789 |
| 2 | 1.60357 | 14.6717 | 14.6717 | 0.0681584 | 0.0681584 | 0 | -22.0779 |
| 3 | 1.96396 | 14.6734 | 14.6734 | 0.0681505 | 0.0681505 | 0 | -21.8817 |
| 4 | 2.26779 | 14.6751 | 14.6751 | 0.0681426 | 0.0681426 | 0 | -21.6893 |
| 5 | 2.53546 | 14.6768 | 14.6768 | 0.0681348 | 0.0681348 | 0 | -21.5004 |
| 6 | 2.77746 | 14.6785 | 14.6785 | 0.0681269 | 0.0681269 | 0 | -21.3149 |
| 7 | 3. | 14.6802 | 14.6802 | 0.068119 | 0.068119 | 0 | -21.1324 |

Fig.9

Fig.10

| $F_{Cyl}$ [D] | $F_{Sph}$ [D] | A_cross @ $0\pi$ [mm^2] | A_cross @ $\pi/2$ [mm^2] | N [a.u.] |
|---|---|---|---|---|
| 16. | 2. | 3.7217 | 3.93267 | 1 |
| 16. | 3. | 3.73818 | 3.05447 | 1 |
| 16. | 4. | 3.7547 | 4.17619 | 1 |
| 16. | 5. | 3.77126 | 4.29783 | 1 |
| 16. | 6. | 3.78786 | 4.4194 | 1 |
| 16. | 7. | 3.80452 | 4.54091 | 1 |
| 16. | 8. | 3.82123 | 4.66235 | 1 |
| 16. | 2. | 4.08982 | 4.29904 | 1 |
| 18. | 3. | 4.1094 | 4.42302 | 1 |
| 18. | 4. | 4.12906 | 4.54692 | 1 |
| 18. | 5. | 4.14878 | 4.67074 | 1 |
| 18. | 6. | 4.16857 | 4.7945 | 1 |
| 18. | 7. | 4.18844 | 4.91819 | 1 |
| 18. | 8. | 4.20839 | 5.04183 | 1 |
| 20. | 2. | 4.45703 | 4.66458 | 1 |
| 20. | 3. | 4.47923 | 4.79031 | 1 |
| 20. | 4. | 4.50153 | 4.91595 | 1 |
| 20. | 5. | 4.52391 | 5.04153 | 1 |
| 20. | 6. | 4.54639 | 5.16706 | 1 |
| 20. | 7. | 4.56897 | 5.29253 | 1 |
| 20. | 8. | 4.59166 | 5.41797 | 1 |
| 22. | 2. | 4.82393 | 5.02987 | 1 |
| 22. | 3. | 4.84841 | 5.15705 | 1 |
| 22. | 4. | 4.873 | 5.28416 | 1 |
| 22. | 5. | 4.8977 | 5.41123 | 1 |
| 22. | 6. | 4.64785 | 5.2646 | 2 |
| 22. | 7. | 4.67277 | 5.39176 | 2 |
| 22. | 8. | 4.49107 | 5.25356 | 3 |
| 24. | 2. | 4.65041 | 4.85552 | 3 |
| 24. | 3. | 4.67688 | 4.9843 | 3 |
| 24. | 4. | 4.70349 | 5.11305 | 3 |
| 24. | 5. | 4.73024 | 5.24178 | 3 |
| 24. | 6. | 4.75713 | 5.3705 | 3 |
| 24. | 7. | 4.78417 | 5.49919 | 3 |
| 24. | 8. | 4.54784 | 5.36589 | 4 |
| 26. | 2. | 4.75578 | 4.95991 | 4 |
| 26. | 3. | 4.78411 | 5.09007 | 4 |
| 26. | 4. | 4.81261 | 5.22022 | 4 |
| 26. | 5 . | 4.84126 | 5.35034 | 4 |
| 26. | 6 . | 4.87009 | 5.48044 | 4 |
| 26. | 7 . | 4.63739 | 5.35025 | 5 |
| 26. | 8 . | 4.66654 | 5.48034 | 5 |
| 28. | 2 . | 4.86456 | 5.06784 | 5 |
| 28. | 3 . | 4.89465 | 5.19931 | 5 |
| 28. | 4 . | 4.92492 | 5.33075 | 5 |
| 28. | 5 . | 4.95538 | 5.46212 | 5 |
| 28. | 6 . | 4.72562 | 5.33431 | 6 |
| 28. | 7 . | 4.75644 | 5.46553 | 6 |
| 28. | 8 . | 4.52756 | 5.33834 | 7 |
| 30. | 2 . | 4.71734 | 4.92026 | 7 |
| 30. | 3 . | 4.74911 | 5.05313 | 7 |
| 30. | 4 . | 4.78108 | 5.18588 | 7 |
| 30. | 5 . | 4.81326 | 5.31841 | 7 |
| 30. | 6 . | 4.84565 | 5.45055 | 7 |
| 30. | 7 . | 4.61921 | 5.32479 | 8 |
| 30. | 8 . | 4.65203 | 5.45572 | 8 |

Fig.11

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- DE 102005028933 A1
- US 2009164008 A1